# EUROPEAN PATENT SPECIFICATION

(11) **EP 2 177 609 B1**
(45) Date of publication and mention of the grant of the patent: **10.12.2014**
(21) Application number: 08777678.7
(22) Date of filing: 27.06.2008
(51) Int. Cl.: C12N 15/09, C12Q 1/02, C12Q 1/68

(54) **MARKER GENE FOR DETECTION OF TUMOR PROMOTER, AND METHOD FOR DETECTION OF TUMOR PROMOTER**
MARKERGEN ZUM NACHWEIS EINES TUMORPROMOTORS UND VERFAHREN ZUM NACHWEIS EINES TUMORPROMOTORS
GÈNE MARQUEUR ET PROCÉDÉ POUR LA DÉTECTION D'UN PROMOTEUR DE TUMEUR

(30) Priority: 28.06.2007 JP 2007170872; 10.08.2007 JP 2007208833
(43) Date of publication of application: 21.04.2010
(73) Proprietor: Nissin Foods Holdings Co., Ltd., Osaka-shi, Osaka 532-8524 (JP)
(72) Inventor: OHNO, Katsutoshi, Osaka-shi Osaka 532-8524 (JP); MAESHIMA, Hideki, Osaka-shi Osaka 532-8524 (JP); YAMADA, Toshihiro, Osaka-shi Osaka 532-8524 (JP)
(74) Representative: Vossius & Partner
(86) International application number: PCT/JP2008/061766
(87) International publication number: WO 2009/001939

(56) References cited:
- WO-A1-00/22168
- WO-A1-2005/024020
- "GeneChip Mouse genome Arrays", ANNOUNCEMENT AFFYMETRIX, XX, XX, 1 January 2004 (2004-01-01), XP002394531,
- KONDRAGANTI SUDHA R ET AL: "Effects of 3-methylcholanthrene on gene expression profiling in the rat using cDNA microarray analyses", CHEMICAL RESEARCH IN TOXICOLOGY, vol. 18, no. 11, November 2005 (2005-11), pages 1634-1641, XP002623674, ISSN: 0893-228X
- ONO K. ET AL.: 'In Vitro Hatsugan Promotion Shiken ni Okeru Marker Idenshi no Tansaku' J. TOXICOL. SCI. vol. 32, no. SUPPL., May 2007, page S101 + ABSTR. NO. O-49
- SHUKLA A. ET AL.: 'Silica-induced activation of c-Jun-NH2-terminal amino kinases, protracted expression of the activator protein-1 proto-oncogene, fra-1, and S-phase alterations are mediated via oxidative stress' CANCER RES. vol. 61, no. 5, 2001, pages 1791 - 1795, XP008129589

### SEQUENCE LISTING

<110> NISSIN SHOKUHIN KABUSHIKI KAISHA
<120> Marker genes for the detection of tumour promoters
<130> P08-73
<150>JP2007-170872
   <151>2007-06-28
<150>JP2007-208833
   <151>2007-08-10
<160> 54
<170> PatentIn version 3.1
<210> 1
   <211> 22
   <212> DNA
   <213> Artificial Sequence
<220>
   <223> Orm1 primer sense
<400> 1
   actccaccca tctaggattc ca 22
<210> 2
   <211> 24
   <212> DNA
   <213> Artificial Sequence
<220>
   <223> Orm1 primer antisense
<400> 2
   gcaaaggttt ctactcctcc ttca 24
<210> 3
   <211> 22
   <212> DNA
   <213> Artificial Sequence
<220>
   <223> Scarb1 primer sense
<400> 3
   gccaagctat agggtcctga ag 22
<210> 4
   <211> 19
   <212> DNA
   <213> Artificial Sequence
<220>
   <223> Scarb1 primer antisense
<400> 4
   gactgggtgg ctggtctga 19
<210> 5
   <211> 21
   <212> DNA
   <213> Artificial Sequence
<220>
   <223> Stmn1 primer sense
<400> 5
   cccacaaaat ggaggctaac a 21
<210> 6
   <211> 21
   <212> DNA
   <213> Artificial Sequence
<220>
   <223> Stmn1 primer antisense
<400> 6
   tccacgtgct tgtccttctc t 21
<210> 7
   <211> 22
   <212> DNA
   <213> Artificial Sequence
<220>
   <223> Rad21 primer sense
<400> 7
   ggtcttcagc gagctcttgc ta 22
<210> 8
   <211> 21
   <212> DNA
   <213> Artificial Sequence
<220>
   <223> Rad21 primer antisense
<400> 8
   cgacacagct caagcaaact g 21
<210> 9
   <211> 25
   <212> DNA
   <213> Artificial Sequence
<220>
   <223> Nup54 primer sense
<400> 9
   agatgcagac ctgttacgag aaatc 25
<210> 10
   <211> 21
   <212> DNA
   <213> Artificial Sequence
<220>
   <223> Nup54 primer antisense
<400> 10
   tcaagtggct aaggccttcc t 21
<210> 11
   <211> 26
   <212> DNA
   <213> Artificial Sequence
<220>
   <223> Junb primer sense
<400> 11
   attgcttctg tagtgctcct taacac 26
<210> 12
   <211> 22
   <212> DNA
   <213> Artificial Sequence
<220>
   <223> Jun primer antisense
<400> 12
   tgcagtctag cctggcactt ac 22
<210> 13
   <211> 22
   <212> DNA
   <213> Artificial Sequence
<220>
   <223> Dmp1 primer sense
<400> 13
   ccagagggac aggcaaatag tg 22
<210> 14
   <211> 20
   <212> DNA
   <213> Artificial Sequence
<220>
   <223> Dmp1 primer antisense
<400> 14
   gcccagctcc tctccagatt 20
<210> 15
   <211> 28
   <212> DNA
   <213> Artificial Sequence
<220>
   <223> Abi1 primer sense
<400> 15
   aaaattctct gacctttaat cctatggt 28
<210> 16
   <211> 22
   <212> DNA
   <213> Artificial Sequence
<220>
   <223> Abi1 primer antisense
<400> 16
   tgcccacatg taaagccatt ac 22
<210> 17
   <211> 25
   <212> DNA
   <213> Artificial Sequence
<220>
   <223> 6530403A03 primer sense
<400> 17
   attgaaaatg acagtgacct gtttg 25
<210> 18
   <211> 26
   <212> DNA
   <213> Artificial Sequence
<220>
   <223> 6530403A03 primer antisense
<400> 18
   ggactttttc ggctattatc ttgatt 26
<210> 19
   <211> 22
   <212> DNA
   <213> Artificial Sequence
<220>
   <223> Slc2a1 primer sense
<400> 19
   tccaactgga cctcaaactt ca 22
<210> 20
   <211> 20
   <212> DNA
   <213> Artificial Sequence
<220>
   <223> Slc2a1 primer antisens
<400> 20
   ccgcacagtt gctccacata 20
<210> 21
   <211> 27
   <212> DNA
   <213> Artificial Sequence
<220>
   <223> Mrpplf3 primer sense
<400> 21
   gccacagaca taaagaaaaa gatcaac 27
<210> 22
   <211> 26
   <212> DNA
   <213> Artificial Sequence
<220>
   <223> Mrpplf3 primer antisense
<400> 22
   tcttcttttc ttcatctcca ttctga 26
<210> 23
   <211> 21
   <212> DNA
   <213> Artificial Sequence
<220>
   <223> Fosl1 primer sense
<400> 23
   ccgaagaaag gagctgacag a 21
<210> 24
   <211> 23
   <212> DNA
   <213> Artificial Sequence
<220>
   <223> Fosll primer antisense
<400> 24
   cgatttctca tcctccaatt tgt 23
<210> 25
   <211> 22
   <212> DNA
   <213> Artificial Sequence
<220>
   <223> Chek1 primer sense
<400> 25
   ccgactttct aagggtgatg ga 22
<210> 26
   <211> 23
   <212> DNA
   <213> Artificial Sequence
<220>
   <223> Chek1 primer antisense
<400> 26
   cgctgagctt ccctttaatc ttc 23
<210> 27
   <211> 21
   <212> DNA
   <213> Artificial Sequence
<220>
   <223> Pik3r5 primer sense
<400> 27
   gcagagtgtg gtcaggtgtg a 21
<210> 28
   <211> 20
   <212> DNA
   <213> Artificial Sequence
<220>
   <223> Pik3r5 primer antisense
<400> 28
   ggtggcaagc tgctcttctc 20
<210> 29
   <211> 17
   <212> DNA
   <213> Artificial Sequence
<220>
   <223> Junb primer sense
<400> 29
   gccctggcag cctgtct 17
<210> 30
   <211> 17
   <212> DNA
   <213> Artificial Sequence
<220>
   <223> Junb primer antisense
<400> 30
   gcgccaaggt gggtttc 17
<210> 31
   <211> 19
   <212> DNA
   <213> Artificial Sequence
<220>
   <223> Vegfa primer sense
<400> 31
   tgcacccacg acagaagga 19
<210> 32
   <211> 22
   <212> DNA
   <213> Artificial Sequence
<220>
   <223> Vegfa primer antisense
<400> 32
   tcgctggtag acatccatga ac 22
<210> 33
   <211> 21
   <212> DNA
   <213> Artificial Sequence
<220>
   <223> Rif1 primer sense
<400> 33
   caggactgtc tccacggatg a 21
<210> 34
   <211> 23
   <212> DNA
   <213> Artificial Sequence
<220>
   <223> Rif1 primer antisense
<400> 34
   gggtatctag ggtcacaggt tca 23
<210> 35
   <211> 23
   <212> DNA
   <213> Artificial Sequence
<220>
   <223> Illrll primer sense
<400> 35
   ctgcaggaaa agagaatcca aac 23
<210> 36
   <211> 21
   <212> DNA
   <213> Artificial Sequence
<220>
   <223> Illrll primer antisense
<400> 36
   ggaaggcatt gtggaatcaa g 21
<210> 37
   <211> 21
   <212> DNA
   <213> Artificial Sequence
<220>
   <223> Phex primer sense
<400> 37
   gccaagagaa atgggaaagc t 21
<210> 38
   <211> 21
   <212> DNA
   <213> Artificial Sequence
<220>
   <223> Phex primer antisense
<400> 38
   agcacaaaac ctgtccttcc a 21
<210> 39
   <211> 21
   <212> DNA
   <213> Artificial Sequence
<220>
   <223> Tfrc primer sense
<400> 39
   ttgaggcaga ccttgcactc t 21
<210> 40
   <211> 22
   <212> DNA
   <213> Artificial Sequence
<220>
   <223> Tfrc primer antisense
<400> 40
   aaagccaggt gtgtatggat ca 22
<210> 41
   <211> 24
   <212> DNA
   <213> Artificial Sequence
<220>
   <223> Zfhx1b primer sense
<400> 41
   gtgacaagac attccagaaa agca 24
<210> 42
   <211> 22
   <212> DNA
   <213> Artificial Sequence
<220>
   <223> Zfhx1b primer antisense
<400> 42
   tggtgtggtc tctttcctgt gt 22
<210> 43
   <211> 20
   <212> DNA
   <213> Artificial Sequence
<220>
   <223> Rad51ap1 primer sense
<400> 43
   tgaaagcaag aggcccaagt 20
<210> 44
   <211> 23
   <212> DNA
   <213> Artificial Sequence
<220>
   <223> Rad51ap1 primer antisense
<400> 44
   aatgcattgc tgctagagtt cct 23
<210> 45
   <211> 27
   <212> DNA
   <213> Artificial Sequence
<220>
   <223> Hells primer sense
<400> 45
   tctagaatta ctgttggatc gaagtga 27
<210> 46
   <211> 22
   <212> DNA
   <213> Artificial Sequence
<220>
   <223> Hells primer antisense
<400> 46
   tccctgtctt ccctttaatt gg 22
<210> 47
   <211> 20
   <212> DNA
   <213> Artificial Sequence
<220>
   <223> Mcm3 primer sense
<400> 47
   cccaggactc ccagaaagtg 20
<210> 48
   <211> 18
   <212> DNA
   <213> Artificial Sequence
<220>
   <223> Mcm3 primer antisense
<400> 48
   gagggccgcc ttaaaagc 18
<210> 49
   <211> 24
   <212> DNA
   <213> Artificial Sequence
<220>
   <223> Orm2 primer sense
<400> 49
   accttacccc caacttgata aatg 24
<210> 50
   <211> 27
   <212> DNA
   <213> Artificial Sequence
<220>
   <223> Orm2 primer antisense
<400> 50
   acagtggtca tctatggtgt gatactc 27
<210> 51
   <211> 23
   <212> DNA
   <213> Artificial Sequence
<220>
   <223> Car13 primer sense
<400> 51
   ttgagagtgt cacgtggatt gtt 23
<210> 52
   <211> 21
   <212> DNA
   <213> Artificial Sequence
<220>
   <223> Car13 primer antisense
<400> 52
   cacaagaggc ttcggaatct g 21
<210> 53
   <211> 21
   <212> DNA
   <213> Artificial Sequence
<220>
   <223> Ccnb1 primer sense
<400> 53
   gcagcacctg gctaagaatg t 21
<210> 54
   <211> 24
   <212> DNA
   <213> Artificial Sequence
<220>
   <223> Ccnb1 primer antisense
<400> 54
   ttcttgacag tcatgtgctt tgtg

## Description

### TECHNICAL FIELD

The present invention relates to the use of a set of marker genes for detection of a tumor promoter, and a method of detecting a tumor promoter. The present invention particularly relates to the use of a set of marker genes that can be used in a method of detecting a tumor promoter by using the expression level of a specific gene in a cultured cell as an index, instead of the observation of focus formation, in a transformation assay for predicting carcinogenicity as a tumor promoter using a cultured cell, and to a method of detecting a tumor promoter using the marker gene expression level as an index.

### BACKGROUND ART

Foods, as well as various substances present in the environment, may have carcinogenicity. Therefore, a method that can quickly predict the carcinogenicity of such substances has been desired.

Various genotoxicity tests are used as simple test methods for detecting carcinogenicity (oncogenicity). However, there are carcinogens (non-mutagenic carcinogenic substances) that cannot be detected by genotoxicity tests. Most of such substances are called tumor promoters.

It has been confirmed that the carcinogenic mechanism principally involves a two-stage process. The first stage is called "tumor initiation", in which DNA is damaged by a genotoxic (mutagenic) substance. The second stage is called "carcinogenetic promotion", which is known as a process in which a tumor promoter (a substance having tumor-promoting activity) enhances the growth of DNA-damaged cells and promotes tumor formation; however, the mechanism of this promotion may vary.

Examples of substances known as tumor promoters include endogenous hormones produced in the body, as well as various organic and inorganic compounds. Tumor promoters are not always genotoxic. Accordingly, and problematically, tumor promoters cannot be always detected by genotoxicity tests.

The action mechanisms of the compounds known as tumor promoters are not identical. Examples of the action mechanisms include cell growth promotion, cytotoxicity, promotion of enzymes such as protein kinase C, and inhibition of enzymes. The detailed molecular mechanism of tumor promotion has yet to be elucidated.

A method generally used for detecting a tumor promoter is a two-stage carcinogenicity test using rodents as a test animal. However, this method necessitates animal breeding facilities, and requires a long test period, such as 8 to 24 weeks or longer. Furthermore, evaluations require autopsies etc., thus requiring a high level of skill and expertise. Accordingly, an *in vitro* test that enables many samples to be tested in a simpler manner has been desired.

On the other hand, examples of *in vitro* test systems currently known include a transformation assay using BALB/c 3T3 cells as cultured cells (Non-Patent Document 1), and a transformation assay using Bhas cells produced by introducing a *v-Ha-Ras* gene into BALB/c 3T3 cells (Non-Patent Document 2). These tests are simpler in operation than animal tests, and are excellent tumor promoter detection methods.

Although these tests are simpler in operation than animal tests, a long test period is required. More specifically, it takes 25 days to complete the BALB/c 3T3 cell transformation assay, and 21 days to complete the Bhas cell transformation assay. Even if the period of cell preparation is excluded from the test period, it takes a long period of about 20 days from the addition of a test substance to the obtaining of results. Furthermore, the evaluation requires microscopic observation of the focus formation ability of cultured cells, thus requiring a high level of skill and expertise.

Other methods currently used for detecting tumor promoters include, for example, metabolic cooperation assays, and tests using EB virus. However, these methods have problematic detection ability and complicated procedures, and are less reliable than the above-mentioned tests.

Along with recent technical developments regarding genomic information, hazard assessments of chemical substances, such as assessments of carcinogenicity, have been performed at the genetic level. In general, carcinogenicity tests using animals require a test period of two years. Even with the use of an animal that is prone to develop cancer, it takes several months to complete the test, and the evaluation requires expertise, such as autopsy expertise. In contrast, evaluation based on gene expression can detect carcinogenicity within several days after the administration of a carcinogen, and can predict carcinogenesis by reading numeric data. Accordingly, this method is advantageous in terms of the shortening of the test period, ease of operation, and the low level of skill required.

There are various levels of gene expression analysis. Global gene expression analysis of tens of thousands of genes using DNA microarrays (Patent Documents 1 and 2) used in, for example, carcinogenicity tests using animals, is an excellent method having the following advantages: thousands to tens of thousands of expressed genes can be analyzed; the expression pattern of the entire gene can be analyzed and compared to detect a phenomenon whose mechanism is unknown, such as carcinogenesis; and unknown genes can also be analyzed. However, using global gene expression analysis to evaluate many samples is difficult, because global gene expression analysis has disadvantages such as the inclusion in the analysis of many genes unnecessary for the evaluation, and the high cost of the test equipment, reagent, and analysis.

Another known method uses marker genes that can predict the onset of a specific disease by global gene analysis or from a known mechanism, and evaluating the expression levels of the specific marker genes by quantitative RT-PCR or like methods (Patent Documents 3 and 4). This method is simple and relatively low in cost, and suitable for screening, i.e., testing many samples.
Non-Patent Document 1: "Short-term two-stage transformation assay using BALB/c 3T3 cells to predict the carcinogenicity of chemical substances", TR Z 0023, Japanese Standards Association, 2002
Non-Patent Document 2: Mutat. Res., vol. 557, 191-202, 2004
Patent Document 1: Japanese Unexamined Patent Publication No. 2007-54022
Patent Document 2: WO2005/024020
Patent Document 3: Japanese Unexamined Patent Publication No. 2006-162446
Patent Document 4: Japanese Unexamined Patent Publication No. 2004-248575
The Affymetrix mouse gene expression array (GeneChip Mouse Genome 430 2.0 Array) which has been used for the experiments described herein is disclosed in Non-Patent Document 3.
In Non-Patent Document 4, 3-Methylcholanthrene, a known carcinogen, is described to cause an over-expresson of the mouse gene Ornm 1.
In Patent Document 5, an assay for detection of gene expression changes after phorbol-myristate acetate (TPA) treatment on cultured mouse keratinocytes and the differently expressed genes identified with this study are disclosed.
Non-Patent Document 3: Announcement of Affymetrix on January 1, 2004
Non-Patent Document 4: Kondraganti et al., Chemical Research in Toxicology, vol. 18(11), 163-1641, 2005.
Patent Document 5: WO 00/11168 A1

### DISCLOSURE OF THE INVENTION

### PROBLEM TO BE SOLVED BY THE INVENTION

An object of the present invention is to find gene(s) that can be used as a marker for detecting tumor promoters in a transformation assay using a cultured cell, which is an *in vitro* test system for predicting carcinogenicity as a tumor promoter. Another object of the present invention is to establish a method for detecting tumor promoters at the genetic level using the marker gene(s). A further object of the present invention is to provide an *in vitro* test system for determining whether a substance is a tumor promoter in a simple manner and within a short period of time, by applying the tumor promoter detection method to a transformation assay using a cultured cell.

### MEANS FOR SOLVING THE PROBLEM

The present invention relates to the use of a set of marker-genes as a marker in a method of detecting a tumor promoting agent using a BALB/c 3T3 cell, a clonal strain thereof, or a transformant thereof, the set of marker-genes comprising at least 7 genes in the following genetic group A-2:
(Gene Symbol)
Orm1
Jun
Plf ; Plf2 ; Mrpplf3
Fosl1
Il1rl1
Hells
Ccnb1
wherein the three genes Plf, Plf2 and Mrpplf3 are assayed as identical.

Furthermore, the invention relates to a method of detecting a tumor promoting agent, comprising the steps of:
bringing a BALB/c 3T3 cell, a clonal strain thereof, or a transformant thereof into contact with a test substance;
determining the expression level of a marker-gene in the cell brought into contact with the test substance;
comparing the determined expression level with the expression level of a control brought into contact with a test substance-free solvent; and
evaluating the test substance as having tumor-promoting activity, when the comparison shows that (i) the sum of a set of marker-genes expression levels or (ii) the number of genes of a set of marker-genes expressed at high levels in the test substance-contacted cells is greater than that of the control,
the set of marker-genes being a set of marker-genes defined in one of the invention.

Use of a kit in a method of detecting a tumor promoting agent using a BALB/c 3T3 cell, a clonal strain thereof, or a transformant thereof, the kit comprising a reagent for determining the set of marker-genes of the invention, wherein said reagent is selected from the group consisting of a reagent for measuring the expression levels by PCR comprising a sense or antisense primer or one or more sets of sense and antisense primers for each marker gene of the invention;
a reagent for northern blotting assay comprising probes for the marker genes of the invention;
a reagent for DNA microarray assay comprising arrays carrying probes for the marker genes of the invention; and a reagent for immunoassay comprising antibodies to transcripts of the marker genes of the invention or microplates on which said antibodies are immobilized.
The present inventors first performed DNA microarray assay to find genes that could be used for evaluating tumor-promoting activity in a transformation assay. Since the mechanism of tumor promotion is complex and for the most part has yet to be elucidated, 9 types of various tumor promoters with different properties and different structures were used to select marker genes.

Marker genes were selected considering the following points:
(1) increased expression of the gene is highly commonly observed with the use of any tumor promoter;
(2) actual increase of the gene expression level upon carcinogenesis can be confirmed in the literature, etc.;
(3) the gene is considered to be closely associated with tumor promotion, such as cell proliferation, oncogene, apoptosis inhibition, and cytoskeletal change;
(4) the gene has a high tumor promoter detection ability, although its function is unknown; and
(5) the expression level of the gene is less than 1.5 times that of the negative control, when a test substance is not a tumor promoter.
As a result, 27 types of genes were selected.

Further, the present inventors performed a transformation assay using a cultured cell, and established a method for detecting a tumor promoter in a simple manner and within a short period of time, without the necessity of actual observation of focus formation in a transformation assay using a cultured cell, the method comprising exposing the cultured cell to a test substance, i.e., a potential tumor promoter, extracting the total RNA including m-RNA from the cultured cell after a certain amount of time has elapsed, and determining the expression level(s) of selected marker gene(s) in the total RNA.

Herein disclosed is the following:
1. A set of marker-genes for use as a marker in a method of detecting a tumor promoter using a BALB/c, a clonal strain thereof, or a transformant thereof, the set of marker-genes comprising at least 7 genes selected from the following genetic group A:
genetic group A

| (Gene Symbol) | (GenBank Accession) |
|---|---|
| Orm1 | NM_008768 |
| Scarb1 | NM_016741 |
| Stmn1 | NM_019641 |
| Rad21 | NM_009009 |
| Nup54 | NM_183392 |
| Jun | NM_010591 |
| Dmp1 | NM_016779 |
| Abi1 | NM_001077190 ; NM_001077192; NM_001077193 ; NM_007380 ; NM_145994 |
| 6530403A03Rik | NM_026382 |
| Slc2a1 | NM_011400 |
| Plf ; Plf2 ; Mrpplf3 | NM_011118 ; NM_011954 ; NM_031191 |
| Fosl1 | NM_010235 |
| Chek1 | NM_007691 |
| Pik3r5 | NM_177320 |
| Junb | NM_008416 |
| Vegfa | NM_001025250 ; NM_001025257 ;NM_009505 |
| Rif1 ; LOC671598 | NM_175238 ; XR_003484 |
| Il1rl1 | NM_001025602 ; NM_010743 |
| Phex | NM_011077 |
| Tfrc | NM_011638 |
| Zfhx1b | NM_015753 |
| Rad51ap1 | NM_009013 |
| Hells | NM_008234 |
| Mcm3 | NM_008563 |
| Orm2 | NM_011016 |
| Car13 | NM_024495 |
| Ccnb1 | NM_172301 |

The invention relates to the use of a set of marker-genes as a marker in a method of detecting a tumor promoting agent using a BALB/c 3T3 cell, a clonal strain thereof, or a transformant thereof, the set of marker-genes comprising at least 7 genes in the following genetic group A-2:
(Gene Symbol)
Orm1
Jun
Plf ; Plf2 ; Mrpplf3
Fosl1
Il1rl1
Hells
Ccnb1
wherein the three genes Plf, Plf2 and Mrpplf3 are assayed as identical.

Said at least 7 genes of the use as provided herein above are listed in the following genetic group A-2: genetic group A-2

| (Gene Symbol) | (GenBank Accession) |
|---|---|
| Orm1 | NM_008768 |
| Jun | NM_010591 |
| Plf ; Plf2 ; Mrpplf3 | NM_011118 ; NM_011954 ; NM_031191 |
| Fosl1 | NM_010235 |
| Il1rl1 | NM_001025602 ; NM_010743 |
| Hells | NM_008234 |
| Ccnb1 | NM_172301 |

wherein the three genes Plf, Plf2 and Mrpplf3 are assayed as identical.

The invention also relates to the use of a set of marker genes as provided above, wherein the set of marker genes comprises at least 11 genes in the following genetic group A-2': genetic group A-2'

| (Gene Symbol) | (GenBank Accession) |
|---|---|
| Ora1 | NN_008768 |
| Jun | NM_010591 |
| Plf ; Pelf2 ; Mrpplf3 | NM_011118; NM_011954 ; NM_031191 |
| Fosl1 | NM_010235 |
| rl1rl1 | NM_001025602 ; NM_010743 |
| Hells | NM_008234 |
| Ccnb1 | NM_172301 |
| SIc2a1 | NM_011400 |
| Phex | NM_011077 |
| Scarb1 | NM_016741 |
| Vegfa | NM_001025250 ; NM_001025257 ; NM_009505 |

The invention also relates to the use of a set of marker genes as provided above, wherein the set of marker genes, which comprises at least 22 genes in the following genetic group A-3:
genetic group A-3

| (Gene Symbol) | (GenBank Accession) |
|---|---|
| Orm1 | NM_008768 |
| Scarb1 | NM_016741 |
| Stmn1 | NM_019641 |
| Nup54 | NM_183392 |
| Jun | NM_010591 |
| Abi1 | NM_001077190 ; NM_001077192, NM_001077193 ; NM_007380 ; NM_145994 |
| Slc2a1 | NM_011400 |
| Plf ; Plf2 ; Mrpplf3 | NM_011118 ; NM_011954 ; NM_031191 |
| Fosl1 | NM_010235 |
| Chek1 | NM_007691 |
| Pik3r5 | NM_177320 |
| Vegfa | NM_001025250 ; NM_001025257 ; NM_009505 |
| Rif1 ; LOC671598 | NM_175238 ; XR_003484 |
| Il1rl1 | NM_001025602 ; NM_010743 |
| Phex | NM_011077 |
| Tfrc | NM_011638 |
| Rad51ap1 | NM_009013 |
| Hells | NM_008234 |
| Mcm3 | NM_008563 |
| Orm2 | NM_011016 |
| Car13 | NM_024495 |
| Ccnb1 | NM_172301 |

Herein described is also a method of detecting a tumor promoter, comprising the steps of:
bringing a BALB/c 3T3 cell, a clonal strain thereof, or a transformant thereof into contact with a test substance;
determining the expression level of a marker-gene in the cell brought into contact with the test substance;
comparing the determined expression level with the expression level of a control brought into contact with a test substance-free solvent; and
evaluating the test substance as having tumor-promoting activity, when the comparison shows that (i) the sum of a set of marker-gene expression levels or (ii) the number of a set of genes expressed at high levels in the test substance-contacted cells is greater than that of the control,
the set of marker-genes being the set of marker-genes defined above.

In particular, a method of detecting a tumor promoter comprising the steps of:
bringing a cultured cell into contact with a tumor initiator;
bringing the tumor initiator-contacted cultured cell into contact with a test substance;
determining the expression level of a marker-gene in the cell brought into contact with the test substance;
comparing the determined expression level with the expression level of a control brought into contact with a test substance-free solvent; and
evaluating the test substance as having tumor-promoting activity, when the comparison shows that (i) the sum of the set of marker-genes expression levels or (ii) the number of the marker-genes in the set expressed at high levels in the test substance-contacted cells is greater than that of the control,
the marker-genes/the set of marker genes being the marker-genes/set of marker genes defined above, and wherein the cultured cell is a BALB/c 3T3, a clonal strain thereof, or a transformant thereof.

The invention also provides for the use of a kit in a method of detecting a tumor promoter using a BALBc 3T3 cell, a clonal strain thereof or a transformant thereof, the kit comprising a reagent for determining the set of marker-genes as provided herein.

In the present specification, the "tumor promoter" refers to a substance having tumor-promoting activity.

"tumor-promoting activity" refers to the action of promoting the proliferation of initiated potential tumor cells (DNA-damaged cells) and malignant transformation of the cells. Most of the non-genotoxic carcinogens are tumor promoters.

Most of the tumor promoters were found in two-stage carcinogenicity tests in rodents or other animal tests. The intensity of the activity of the promoters can be estimated from the lowness of the tumor promoter concentration, as well as from the number of tumors and precancerous lesions. For example, when a test substance can cause a specific lesion at a lower concentration, the test substance is evaluated as having a higher tumor-promoting activity. When test substances are used at the same concentration, a test substance is evaluated as having a higher tumor-promoting activity when the number of tumors and precancerous lesions developed is greater.

More specifically, a test substance is evaluated as having tumor-promoting activity when a large number of focus formation and/or a low concentration is the result of a transformation assay using BALB/c 3T3 cells, as shown in the Examples below.

### 1. Marker-gene

The marker-gene as comprised in the set of marker genes to be used in accordance with the invention as characterized in the claims of the present invention, comprises at least one gene selected from the genes described below.

In this specification, the gene refers to a source from which a genetic trait is expressed. Examples of the gene include isolated DNA molecules, RNA molecules, and DNA transcripts.

In this specification, the marker-gene refers to a single gene or a set of genes used as a marker, and can be paraphrased as a marker gene or a set of marker genes.

The base sequences of the genes shown below can be identified by their Accession Nos. in a known database (NCBI).

| | |
|---|---|
| Orm1 | NM_008768 |
| Scarb1 | NM_016741 |
| Stmn1 | NM_019641 |
| Rad21 | NM_009009 |
| Nup54 | NM_183392 |
| Jun | NM_010591 |
| Dmp1 | NM_016779 |
| Abi1 | NM_001077190 ; NM_001077192; NM_001077193 ; NM_007380 ; NM_145994 |
| 6530403A03Rik | NM_026382 |
| Slc2a1 | NM_011400 |
| Plf ; Plf2 ; Mrpplf3 | NM_011118 ; NM_011954 ; NM_031191 |
| Fosl1 | NM_010235 |
| Chek1 | NM_007691 |
| Pik3r5 | NM_177320 |
| Junb | NM_008416 |
| Vegfa | NM_001025250 ; NM_001025257 ; NM_009505 |
| Rif1 ; LOC671598 | NM_175238 ; XR_003484 |
| Il1rl1 | NM_001025602 ; NM_010743 |
| Phex | NM_011077 |
| Tfrc | NM_011638 |
| Zfhx1b | NM_015753 |
| Rad51ap1 | NM_009013 |
| Hells | NM_008234 |
| Mcm3 | NM_008563 |
| Orm2 | NM_011016 |
| Car13 | NM_024495 |
| Ccnb1 | NM_172301 |

All the NM_001077190, NM_001077192, NM_001077193, NM_007380, and NM_145994 are transcript variants of Abi1. Both the DNA microarray assay and the RT-PCR probe assay regard these five genes as identical.

More specifically, Abi1 refers to a gene that can be represented by the sequence of NM_001077190, NM_001077192, NM_001077193, NM_007380, or NM_145994.

NM_011118, NM_011954, and NM_031191 represent three genes, Plf2, Mrpplf3, and Plf. These genes have recently become known as members of prolactin family 2, subfamily c (prl2c) under the names of prl2c3, prl2c4, and prl2c2; and have nearly identical sequences. Both the DNA microarray assay and the RT-PCR probe assay regard these three genes as identical.

More specifically, Plf2, Mrpplf3, and Plf refer to genes that can be represented by the sequence of NM_011118, NM_011954, or NM_031191.

NM_001025250, NM_001025257, and NM_009505 are transcript variants of Vegfa. Both the DNA microarray assay and the RT-PCR probe assay regard these three genes as identical.

More specifically, Vegfa refers to a gene that can be represented by the sequence of NM_001025250, NM_001025257, or NM_009505.

NM_175238 and XR_003484 represent two genes, Rif1 and LOC671598. LOC671598 is a homologue of Rif1. These genes have nearly identical sequences. Both the DNA microarray assay and the RT-PCR probe assay regard these genes as identical.

More specifically, Rif1 and LOC671598 refer to genes that can be represented by the sequence of NM_175238 or XR_003484.

NM_001025602 and NM_010743 are transcript variants of Il1rl1. Both the DNA microarray assay and the RT-PCR probe assay regard these two genes as identical.

More specifically, Il1rl1 refers to a gene that can be represented by the sequence of NM_001025602 or NM_010743.

These marker-gene was found by a method comprising adding substances known as tumor promoters to initiated cells, and performing global gene expression analysis by DNA microarray assay using the total RNA extracted from the cells after a certain amount of time has elapsed, in a transformation assay using BALB/c 3T3 cells.

In particular, to find the marker-gene that can detect all the tumor promoters, and considering the fact that the mechanism of tumor promotion has yet to be elucidated, 9 types of tumor promoters having different properties were selected to perform DNA microarray analysis.

More specifically, the following compounds were selectively used as tumor promoters that are able to form foci in transformed cells, and that have no genetic toxicity:
(i) TPA, which is a typical tumor promoter and is a phorbol ester capable of activating protein kinase C;
(ii) okadaic acid, which has protein phosphatase 1,2A-inhibitory activity;
(iii) phenobarbital sodium, which activates various drug-metabolizing enzymes;
(iv) saccharin sodium, which causes bladder cancers at high concentrations;
(v) sodium orthovanadate, which is a vanadic acid salt having protein tyrosine phosphatase-inhibitory activity;
(vi) lithocholic acid, which is a secondary bile acid derived from an organism; and
(vii) insulin, which promotes cell growth.
   As tumor promoters having genetic toxicity, the following substances were selectively used:
(viii) zinc chloride, which increases metallothionein; and
(ix) sodium arsenite, which varies.in toxicity induced by the inhibition of oxidative phosphorylation.

The 9 types of tumor promoters having different properties and different structures were individually added to test systems. RNA was extracted from the cells, and comprehensive analysis of the expressed genes was performed using DNA microarrays.

The RNA extraction time was set to 48 hours after the addition of the test substance, in order to exclude the influence of the expression of drug-metabolizing enzyme genes that have little to do with test substance-specific carcinogenicity promotion. The mechanism of tumor promotion is complex, and for the most part has yet to be elucidated. Therefore, the marker-gene was selected from about 40,000 types of genes on a DNA microarray according to the following rules.

As genes whose expression levels are highly reliable according to a fixed method, and whose expression is increased with the use of one of the tumor promoters, about 6,700 types of genes were first selected. More specifically, as genes (1) whose expression levels were up-regulated by more than 1.5-fold compared to the negative control in at least one of the test systems containing a tumor promoter, about 6,700 types of genes were selected.

Subsequently, as genes whose increased expression is highly commonly observed in all the tumor promoters, 325 types of genes were selected. More specifically, as genes (2) whose expression levels are up-regulated by more than 1.5-fold compared to the negative control in at least five of the test systems containing a tumor promoter, 325 types of genes were selected.

Further screening was performed to select the following genes: (a) genes whose actual increase in the expression level upon carcinogenesis can be confirmed in the literature, etc.; (b) genes that are considered to be closely associated with the properties of tumor promotion, such as cell proliferation, oncogene, apoptosis inhibition, and cytoskeletal change; (c) genes that have high tumor promoter detection ability, although their functions are unknown; and (d) genes whose expression levels are less than 1.5 times that of the negative control, when a test substance is not a tumor promoter.

More specifically, as genes (3) whose expression levels are not more than 1.25 times that of the negative control in a tumor promoter-free test system, 98 genes were selected from the genes (2). Further, as genes (4)(i) whose expression levels are sufficiently high, and as genes (4)(ii) that are considered to be closely associated with the properties of carcinogenesis or tumor promotion, such as cell proliferation, oncogene, apoptosis inhibition, and cytoskeletal change, 27 types of genes were selected from the genes (3).

As a result, 27 types of genes were selected. Table 1 shows principal functions of the genes.

**[Table 1]**

| Reference Sequence | Gene Symbol | Gene functions |
|---|---|---|
| NM_008768 | 0rm1 | Acute phase reactive protein |
| NM_016741 | Scarb1 | adhesion |
| NM_019641 | Stmn1 | Microtubuledepolymerbation |
| NM_009009 | Rad21 | DNA repair |
| NM_183392 | Nup54 | Transportation |
| MM_010591 | Jun | Oncogene |
| NM_016779 | Dmp1 | Cell surface modification |
| NM_001077190 ; NM_001077192 ; NM_001077193 ; NM_007380 ; NM_145994 | Abi1 | Oncogene-related |
| NM_026382 | 6530403A03Rik | Unknown gene |
| NM_011400 | Sic2a1 | Sugar transport |
| NM_011118 ; NM_011954 ; NM_031191 | Plf : Plf2 ; Mrpplf33 | Cell division |
| NM_010235 | Fosl1 | Oncogene-related |
| NM_007691 | Chek1 | DNA damage |
| NM_177320 | Pik3r5 | P13 kinase |
| NM_008416 | Junb | Oncogene-retated |
| NM_001025250 : NM_001025257 ; NM_009505 | Vegfa | Neovascularization |
| NM_175238 ; XR_003484 | Rif1 ; LOC671598 | Telomere maintenance |
| NM_001025602 ; NM_010743 | Il1rl1 | DNA methylation |
| NM_011077 | Phex | Phosphorylation-related |
| NM_011638 | Tfrc | Transferrin uptake |
| NM_015753 | 2fhx1b | Zinc finger |
| NM_009013 | Rad51ap1 | DNA repair |
| NM_008234 | Hells | Apoptosis inhibition |
| NM_008563 | Mcm3 | DNA replication |
| NM_011016 | 0rm2 | Acute phase reactive protein |
| NM_024495 | Car13 | Carbon metabolism |
| NM_172301 | Ccnb1 | Cell cycle |

The marker-genes to be employed in context of this invention refers to genes as shown in Table 1, i.e., a set of genes selected from genetic group A shown below, wherein the present invention relates to the use of a set of marker-genes as a marker in a method of detecting a tumor promoting agent using a BALB/c 3T3 cell, a clonal strain thereof, or a transformant thereof, the set of marker-genes comprising at least 7 genes in the following genetic group A-2:
(Gene Symbol)
Orm1
Jun
Plf ; PIf2 ; Mrpplf3
Fosl1
Il1rl1
Hells
Ccnb1
wherein the three genes Plf, Plf2 and Mrpplf3 are assayed as identical.

More specifically, the marker-gene as described herein may be a full set of 27 types of genes belonging to genetic group A, a set of several genes selected from genetic group A, or one gene selected from genetic group A.
Genetic group A:

| (Gene Symbol) | (GenBank Accession) |
|---|---|
| Orm1 | NM_008768 |
| Scarb1 | NM_016741 |
| Stmn1 | NM_019641 |
| Rad21 | NM_009009 |
| Nup54 | NM_183392 |
| Jun | NM_010591 |
| Dmp1 | NM_016779 |
| Abi1 | NM_001077190 ; NM_001077192; NM_001077193 ; NM_007380 ; NM_145994 |
| 6530403A03Rik | NM_026382 |
| Slc2a1 | NM_011400 |
| Plf ; Plf2 ; Mrpp1f3 | NM_011118 ; NM_011954 ; NM_031191 |
| Fosl1 | NM_010235 |
| Chek1 | NM_007691 |
| Pik3r5 | NM_177320 |
| Junb | NM_008416 |
| Vegfa | NM_001025250 ; NM_001025257 ; NM_009505 |
| Rifl ; LOC671598 | NM_175238 ; XR_003484 |
| Il1rl1 | NM_001025602 ; NM_010743 |
| Phex | NM_011077 |
| Tfrc | NM_011638 |
| Zfhx1b | NM_015753 |
| Rad5ap1 | NM_009013 |
| Hells | NM_008234 |
| Mcm3 | NM_008563 |
| Orm2 | NM_011016 |
| Car13 | NM_024495 |
| Ccnb1 | NM_172301 |

An example of the set of marker-genes for use in context of the present invention is a gene set A-2 comprising at least all the 7 genes in the following genetic group A-2:

| (Gene Symbol) | (GenBank Accession) |
|---|---|
| Orm1 | NM_008768 |
| Jun | NM_010591 |
| Plf ; Plf2 ; Mrpplf3 | NM_011118 ; NM_011954 ; NM_031191 |
| Fosl1 | NM_010235 |
| Il1rl1 | NM_001025602 ; NM_010743 |
| Hells | NM_008234 |
| Ccnb1 | NM_172301 |

Gene set A-2 may consist of the above 7 genes, or may further include one or more genes selected from genetic group A.

A further example of the set of marker-genes for use in context of the present invention is a gene set A-2' comprising at least all the 11 genes in the following genetic group A-2':

| (Gene Symbol) | (GenBank Accession) |
|---|---|
| Orm1 | NM_008768 |
| Jun | NM_010591 |
| Plf ; Plf2 ; Mrpplf3 | NM_011118 ; NM_011954 ; NM_031191 |
| Fosl1 | NM_010235 |
| Il1rl1 | NM_001025602 ; NM_010743 |
| Hells | NM_008234 |
| Ccnb1 | NM_172301 |
| Slc2a1 | NM_011400 |
| Phex | NM_011077 |
| Scarb1 | NM_016741 |
| Vegfa | NM_001025250 ; NM_001025257 ; NM_009505 |

Gene set A-2' may consist of the above 11 genes, or may further include one or more genes selected from genetic group A.

Another example of the set of marker-genes for use in context of the present invention is a gene set A-3 comprising at least all the 22 genes in the following genetic group A-3:

| (Gene Symbol) | (GenBank Accession) |
|---|---|
| Orm1 | NM_008768 |
| Scarb1 | NM_016741 |
| Stmn1 | NM_019641 |
| Nup54 | NM_183392 |
| Jun | NM_010591 |
| Abi1 | NM_001077190 ; NM_001077192; NM_001077193 ; NM_007380 ; NM_145994 |
| Slc2a1 | NM_011400 |
| Plf ; Plf2 ; Mrpplf3 | NM_011118 ; NM_011954 ; NM_031191 |
| Fosl1 | NM_010235 |
| Chek1 | NM_007691 |
| Pik3r5 | NM_177320 |
| Vegfa | NM_001025250 ; NM_001025257; NM_009505 |
| Rif1 ; LOC671598 | NM_175238 ; XR_003484 |
| Il1rl1 | NM_001025602 ; NM_010743 |
| Phex | NM_011077 |
| Tfrc | NM_011638 |
| Rad51ap1 | NM_009013 |
| Hells | NM_008234 |
| Mcm3 | NM_008563 |
| Orm2 | NM_011016 |
| Car13 | NM_024495 |
| Ccnb1 | NM_172301 |

The gene set A-3 may consist of the above 22 genes, or may further include one or more genes selected from genetic group A.

The marker-genes as disclosed herein can be used as a marker in a tumor promoter detection method using a cultured cell.

An example of the tumor promoter detection method using a cultured cell is described below in Section 2 ("Tumor promoter detection method").

More specifically, the marker-genes as disclosed herein can be used, as described later, in a method comprising exposing a cultured cell to a test substance (a tumor promoter), extracting the total RNA from the cultured cell after a certain elapsed time, and measuring the expression level of m-RNA contained in the total RNA.

Further, the marker-genes as disclosed herein can be used in a method of quantitatively determining the expression level of a marker gene-encoded protein in a cultured cell using an antibody specific for the protein encoded by the marker gene. For example, various enzyme immunoassays, radioimmunoassays, solid phase enzyme immunoassays, etc. can be used. The antibody may be a polyclonal or monoclonal antibody.

### 2. Tumor promoter detection method

The present invention provides for a method of detecting a tumor promoter from the gene expression level using the set of marker-genes mentioned above.

The detection method of the present invention comprises the steps of:
bringing a BALB/c 3T3 cell, a clonal strain thereof, or a transformant thereof into contact with a test substance;
determining the expression level of a marker-gene in the cell brought into contact with the test substance;
comparing the determined expression level with that of a control brought into contact with a test substance-free solvent; and
evaluating the test substance as having tumor-promoting activity, when the comparison shows that (i) the sum of a set of marker-genes expression levels or (ii) the number of genes of the set of marker-genes expressed at high levels in the test substance-contacted cells is greater than that of the control, wherein the set of marker genes is the set of marker genes defined herein and in the claims as A-2, A-2' and/or A3.

### (1) Cell to be used

Various types of cultured cells used in general transformation assays can be used as the cultured cell.

Since mouse genes were used in the DNA microarray assay for selecting the marker-gene in the present invention, the cell is preferably a mouse-cultured cell, i.e., a mouse-derived cell.

In context of this invention, the cell is a BALB/c 3T3 cell, i.e., a BALB/c mouse embryonic fibroblast cell line, clonal strains thereof, and transformants thereof, such as Bhas cells obtained by introducing a v-Ha-Ras gene into BALB/c 3T3 cells.

### (2) Test substances

The kind of test substance is not particularly limited. The test substance may be a low molecular or high molecular compound, or may be a mixture of different kinds of substances, such as foods, processed foods, wastes, and incinerated waste.

As the positive control, for example, TPA (phorbol 12-myristate 13-acetate), i.e., a potent tumor promoter, can be used.

As the negative control, a solvent alone is typically used. In the present specification, the "negative control" refers to a control cell sample brought into contact with a test substance-free solvent, unless otherwise specified.

### (3) Determination of the expression level

Examples of the method of determining the expression level include, but are not limited to, a method comprising amplifying cDNA obtained by reverse transcription of mRNA and performing quantitative RT-PCR using marker gene(s) as the target; a northern blotting method comprising directly determining the mRNA level using a probe; and a method of analyzing the expression level of mRNA using a DNA microarray carrying marker gene(s). Any of such methods can be used. The quantitative RT-PCR methods are particularly preferable in view of their low operational costs and their ability to obtain test results in several hours.

The mRNA can be extracted according to a known method. For example, each test substance is added and allowed to stand for a certain period, typically 36 to 72 hours, after which the medium of each test substance-added group was extracted. After washing with PBS, the total RNA is extracted and the expression level of m-RNA of the marker-gene contained in the total RNA is determined. For the extraction of the total RNA, a DNase treatment is preferably performed.

### (4) Comparison of the expression level

The marker-gene expression levels determined in (3) is compared with that of the control cell brought into contact with a test substance-free solvent, i.e., the negative control. For the comparison, an appropriate standard curve may be prepared according to a usual method, and the expression level may be normalized using an internal standard gene. The expression level of a positive control may also be determined, whereby a comparison to the negative control can be combined with a comparison to the positive control.

For example, a standard curve is prepared using a dilution series of cDNA of the negative or positive control, and the expression level relative to the standard curve is calculated. The calculated expression level is normalized by the expression level of β-actin used as an internal standard gene. The normalized expression level is divided by that of the negative control group. The expression level of each gene is calculated as an expression level relative to that of the negative control group, thus obtaining the expression level of each marker gene. A comparison of the obtained expression level(s) of the marker gene(s) with that of the negative control provides comparative results of the expression level(s) of the marker gene(s) in test substance-added systems.

### (5) Evaluation of tumor promoters

Based on the comparative results obtained in (4), the test substance is evaluated on whether the test substance has tumor-promoting activity, i.e., whether the test substance is a tumor promoter. More specifically, when (i) the sum of marker-gene expression levels or (ii) the number of genes of the marker-gene expressed at high levels in the cells brought into contact with a test substance is greater than that of the control, the test substance is evaluated as having tumor-promoting activity.

The criteria by which the test substance is evaluated as a tumor promoter can be appropriately selected according to various methods for analyzing the expression level of the marker-gene for detection of tumor promoters.

To analyze the expression of the marker-gene for detection of the tumor promoters, for example, (1) analysis of the number of genes of the marker-gene up-regulated by more than 1.5-fold compared to the negative control, (2) analysis of the sum of the marker-gene expression levels, (3) cluster analysis, etc. can be used. Methods involving the multiplication of other coefficients are also usable.

More specifically, when the sum of marker-gene expression levels in cells brought into contact with a test substance is greater than that of the negative control, i.e., greater than the sum of marker-gene expression levels in control cells brought into contact with a test substance-free solvent, the test substance can be evaluated as having tumor-promoting activity.

Further, the expression level of each of the marker gene(s) in the cell brought into contact with a test substance is compared with that of the negative control cell. When there are a great number of genes of the marker-gene whose expression levels in test substance-contacted cells are up-regulated by more than 1.5-fold compared to the negative control, the test substance is evaluated as having tumor-promoting activity.

The intensity of the tumor promoters can also be evaluated from the above results. More specifically, the greater the sum of the marker-gene expression levels in the test substance-contacted cells, compared to that of the negative control, the more the test substance can be evaluated as having potent tumor promoter activity.

The greater the number of genes of the marker-gene whose expression levels in the test substance-contacted cells are up-regulated by more than 1.5-fold compared to the negative control, the more the test substance can be evaluated as having potent tumor promoter activity.

The method of the present invention may further include other steps, if necessary. For example, the method may comprise the step of bringing cultured cells into contact with a tumor initiator.

More specifically, in a transformation assay using a cultured cell, two or more substances known as tumor promoters are added to an initiated cell, RNA is extracted from the cell after a certain period of time has elapsed, and the expression level of each marker gene is determined.

The type of marker gene can be selected according to the type and structure of the test substance (tumor promoter), as well as the type of the cultured cell.

For example, a tumor promoter can be detected by determining the expression level of one of the marker genes and using the determined expression level as an index.

Particularly when BALB/c 3T3 cells is used, tumor promoters can be detected by using the expression level of Orm1 gene (NM_008768) as an index, irrespective of the type and structure of the test substance. However, since the Orm1 gene is expressed at low levels in Bhas cells, this gene cannot be used in Bhas cells.

When using BALB/c 3T3 cells, the marker-gene preferably comprises at least Orm1 gene (NM_008768), particularly preferably comprises a gene belonging to genetic group A-2, and more preferably a gene belonging to genetic group A-3. When using Bhas cells, the marker-gene preferably comprises at least Fosll (NM_010235), Hells (NM_008234), and Ccnb1 (NM_172301), more preferably a gene belonging to generic group A-2, and even more preferably genes belonging to generic group A-3.

The expression levels of two or more genes can also be used as an index to detect the tumor-promoting activity.

For example, when the sum of the expression levels of two or more marker genes is used as an index, a method of using at least one of Scarb1 (NM_016741), Stmn1 (NM_019641), Plf; Plf2; Mrpplf3 (NM_011118; NM_011954; NM_031191), Fosll (NM_010235), and Illrll (NM_001025602; NM_010743) as the marker-gene is preferable.

A more preferable method is using as an index the expression levels of all the 27 types of genes shown in genetic group A to detect tumor promoters.

The number of marker genes to be used may vary depending on the type and structure of the test substance used. In general, to assess the intensity of tumor-promoting activity as a tumor promoter, all the marker genes, or as many marker genes as possible when using some of the marker genes as an index are preferably used.

If necessary, the evaluation can be made in combination with other results, such as analysis results of the expression levels of genes other than the marker genes of the present invention, and analysis results of other physical properties of test substances.

### (6) Use in the transformation assay using cultured cells for predicting carcinogenicity as a tumor promoter

The detection method using the marker-gene according to the present invention can be used as a method of detecting a tumor promoter by determining changes in the expression levels of a gene in cultured cells, instead of observation of focus formation in a conventional transformation assay for predicting carcinogenicity as a tumor promoter.

The "transformation assay for predicting carcinogenicity as a tumor promoter" as referred to herein is, for example, a method described in "Short-term two-stage transformation assay using BALB/c 3T3 cells to predict the carcinogenicity of chemical substances", TR Z 0023, published by Japanese Standards Association, 2002. More specifically, the method comprises exposing cultured cells to a tumor initiator, and culturing the cells for a specific period; exposing the cells to a test substance whose tumor-promoting activity is to be examined, and culturing the cells for about 20 days; and staining the cells, and observing and counting the foci (cell clumps) formed in culture dishes to calculate the transformation frequency, and thereby predict the tumor-promoting activity.

The transformation assay generally comprises the following steps. After cells are seeded into 60 mm cell culture dishes or plates and subjected to tumor initiation, the cells are cultured for several days. When cells not necessarily requiring tumor initiation are used, the cells are seeded and cultured for several days. Subsequently, the medium is replaced with a medium containing a test substance, and the cells are cultured typically for about 20 days and then stained. Foci (cell clumps) formed in culture dishes are observed and counted to calculate the transformation frequency.

According to the present invention, the focus formation in the above method is replaced by the following steps. After cells are cultured for a certain period and exposed to a test substance whose tumor-promoting activity is to be examined, the cells are cultured for about 36 to about 72 hours, after which RNA is extracted from the cells. Using a marker-gene as the target gene, the expression level of the marker-gene is determined and used as an index to detect a tumor promoter.

More specifically, instead of the following steps in a conventional transformation method using cultured cells for predicting carcinogenicity as a tumor promoter:
culturing cells for a specific period; exposing the cultured cells to a test substance whose tumor-promoting activity is to be examined; culturing the cells for about 20 days, and then staining the cells; and observing and counting the foci (cell clusters) formed in culture dishes,
the method of the present invention comprises the following steps:
   culturing cells for a specific period; exposing the cultured cells to a test substance whose tumor-promoting activity is to be examined; culturing the cells for about 36 to about 72 hours, and then extracting RNA from the cells; and determining the expression level of a marker-gene as the target,
   whereby the method as disclosed herein can detect tumor promoters at the gene expression levels.

### (7) Test for predicting carcinogenicity as a tumor promoter

The conventional transformation assay using cultured cells requires about 20 days of culturing to form foci after exposing initiated cells to a test substance (a tumor promoter).

In contrast, when using the tumor promoter detection method of the present invention, a tumor promoter can be detected within about 3 to 4 days after exposure to a test substance (a tumor promoter). Thus, a test for predicting carcinogenicity as a tumor promoter can be performed within a short period of time.

The following method can be mentioned as an example:
a test method for predicting the carcinogenicity of a test substance as a tumor promoter, the method comprising the steps of
   1) bringing cultured BALB/c 3T3 cells into contact with a tumor initiator;
   2) bringing the tumor initiator-contacted cells into contact with a test substance;
   3) culturing the test substance-contacted cells for a specific period, and then extracting RNA;
   4) subjecting the RNA extracted in step (3) to PCR using a primer or a set of primers as shown in Table 2, and determining the marker-gene expression levels in the test substance-contacted cells;
   5) comparing the determined expression levels with the expression levels in control cells brought into contact with a test substance-free solvent; and
   6) evaluating the test substance as a tumor promoter when (i) the sum of the marker-gene expression levels or (ii) the number of genes of the marker-gene expressed at high levels in the test substance-contacted cells is greater than that of the control, the marker-gene being the set of marker-genes described above.

Although any known tumor initiator can be used as the tumor initiator, MCA (3-methylcholanthrene) is typically used.

Although the cell-culturing period in step 3 can be suitably selected, the period is typically about 36 to about 72 hours.

The PCR primer used for detecting the marker-gene in step 4 is not limited to those of the sequences shown in Table 2. Any other appropriately designed primer may also be used.

More specifically, the following procedures can be mentioned as an example of the steps of the method; however, the examples should not be construed as limitative of the present invention.

### 1) Cell seeding (Day 0)

BALB/c 3T3 cells in the logarithmic growth phase are seeded into a predetermined number of culture dishes in an amount of 1.0 x 10⁴ cells per culture dish using MEM medium containing 10% FBS (hereinafter referred to as a test medium).

### 2) MCA treatment (Day 1)

After culturing in a carbon dioxide incubator for 24 hours, MCA is added as a tumor initiator.

### 3) Medium replacement (Day 4)

72 hours after the addition of MCA, the medium is aspirated from each culture dish, and replaced with 5 ml each of normal medium or DME/F12 medium containing ITES and 2% FBS (hereinafter referred to as a test medium).

### 4) Test substance treatment (the first time) (Day 7)

The medium is aspirated from the culture dishes of each group. 5 ml of a medium containing a predetermined amount of a test substance is added to each test substance-added group, whereas 5 ml of medium containing TPA is added to a TPA-added group, and 5 ml of medium containing a solvent is added to a solvent-added group.

### 5) RNA extraction

After the test substance treated cells in the culture dishes are cultured in a carbon dioxide incubator for 36 to 72 hours, RNA is extracted. A commercially available extraction kit can be used to extract the RNA.

### 6) Quantitative RT-PCR analysis of marker-gene expression

To examine the expression of a marker-gene using the total RNA obtained in the above step 5), a quantitative RT-PCR is performed using primers for the marker-gene for detection of tumor promoters shown in Table 2 below and using a Real-Time PCR System. The PCR primers used for detecting the genes shown in Table 1 are not limited to those of the sequences shown in Table 2. Any other appropriately designed primer may be used.

To perform the quantitative RT-PCR, RNA extracted from each test substance-added group is first subjected to a reverse transcription reaction to obtain cDNA.

Subsequently, real-time PCR is performed using primers for the marker-gene for detection of tumor promoters shown in Table 2, and dissociation curve analysis is performed. A standard curve is prepared using a dilution series of cDNA of a negative or positive control. The expression level relative to the standard curve is calculated. The calculated expression level is normalized by the expression level of β-actin as an internal standard gene. The normalized expression level is divided by the expression level of the negative control group, whereby the expression level of each gene can be obtained as an expression level relative to the negative control group. The expression levels of the genes shown in Table 1 can be obtained from the thus-obtained expression levels. The marker-gene expression levels can be examined by comparison of the thus-obtained expression levels with that of the negative control.

### 7) Evaluation of carcinogenicity as a tumor promoter

Based on the comparative results of the gene expression levels, the test substance is evaluated as having tumor-promoting activity when the sum of marker-gene expression levels in test substance-contacted cells is greater than that of the negative control. Further, based on a comparison of the expression level of each gene, the test substance is evaluated as having tumor-promoting activity when the number of genes of the marker-gene whose expression levels in test substance-contacted cells is up-regulated by more than 1.5-fold compared to the negative control is great, compared to the negative control.

The tumor-promoting activity of the test substance can also be evaluated by using other test results in combination, such as a transformation assay using focus formation.

The above test can also be used as a test for predicting focus formation in a transformation assay using cultured cells.

### 5. Tumor promoter detection kit

The kit of the present invention includes a reagent for measuring the expression level of a marker-gene.

Examples of the reagent for measuring the expression levels by PCR include a primer or a set of primers. More specifically, the reagent may be a sense or antisense primer for each marker gene shown in Table 2, or one or more sets of sense and antisense primers.

Examples of the reagent for northern blotting assay include probes for the marker genes.

Examples of the reagent for DNA microarray assay include arrays carrying probes for the marker genes.

Examples of the reagent for immunoassay include antibodies to transcripts of the marker genes, microplates on which the antibodies are immobilized, etc.

The kit of the present invention may include other reagents or components than the reagent for measuring the expression levels of the marker genes, as long as they do not impair the object of the present invention. For example, the kit may include a set of primers or probes for an internal standard gene, such as β-actin, GAPDH, or 18srRNA, or antibodies thereto. Further, the kit may include TPA as a positive control, enzymes, buffers, fluorescent reagents used for detection, etc.

### EFFECT OF THE INVENTION

The present invention provides a method or a test system for detecting a tumor promoter in a simple manner within a short period of time and at low cost, the method using a marker-gene and thus obviating the need for long-term culturing and observation of focus formation in a conventional method, i.e., an *in vitro* test system using cultured cells.

The present invention provides a set of marker-genes that can be utilized in a method of detecting a tumor promoter by determining changes in the expression level of a specific gene in cultured cells, instead of observing focus formation in a transformation assay for predicting carcinogenicity as a tumor promoter using cultured cells.

The tumor promoter can be detected by examining the expression level of the set of marker-genes disclosed herein.

When the tumor promoter detection method of the present invention is applied to a conventional transformation assay using cultured cells, the test can be performed in a simple manner without the need for an expensive measuring apparatus, microscopic observation, autopsy, i.e., without requiring experts having skill and expertise in pathological examination. Furthermore, the number of days required for the test, which is about 20 days after addition of the test substance in the conventional method, can be greatly reduced to about 3 to about 4 days.

### BRIEF DESCRIPTION OF THE DRAWINGS

The abbreviations in the Figures stand for the following:
control or cont: negative control, VitCNa: sodium ascorbate, TBHQ: t-butylhydroquinone, As: sodium arsenite, Ins: insulin, LA: lithocholic acid, PB: phenobarbital sodium, NaVO: sodium orthovanadate, ZnCl or Zn: zinc chloride, TPA: phorbol 12-myristate 13-acetate, SS: saccharin sodium, OK: okadaic acid, Per: perylene, BA: benz[a]anthracene, Chr: chrysene, 1-NN: 1-nitronaphthalene, Nap: naphthalene, MNNG: N-methyl-N'-nitro- N-nitrosoguanidine, Mannit: D-mannitol, Menth: DL-menthol, Pro: progesterone, TGFβ1: transforming growth factor, SDM: sulfadimethoxine, KA: kojic acid, BHA: butylhydroxyanisol, Atz: atrazine, VitE: DL-α-tocopherol, 1NP: 1-nitropyrene, Phorb: phorbol, Eug: eugenol, PG: propyl gallate, Cys: L-cysteine hydrochloride, and Phen: phenacetin.

[Fig. 1]

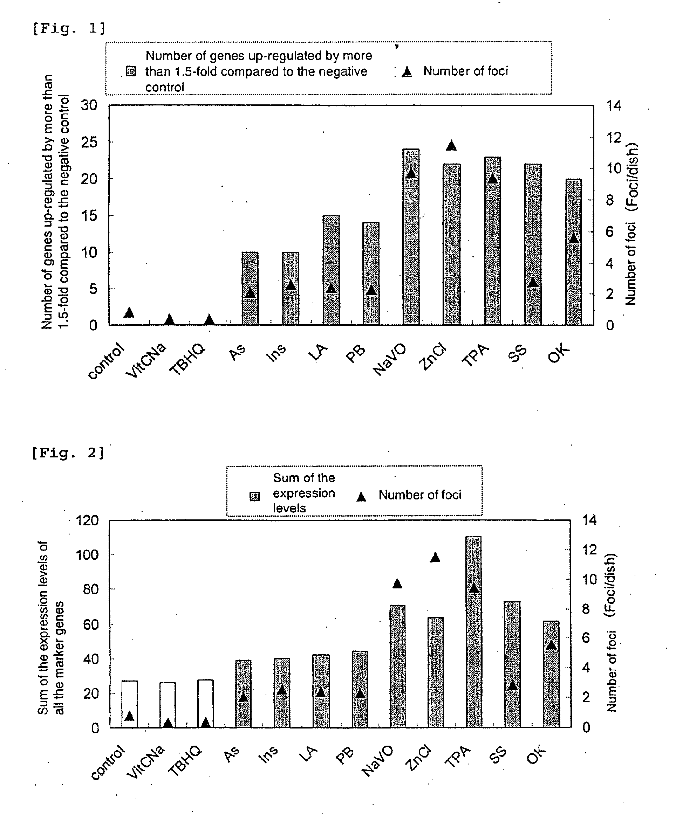

   Fig. 1 shows a relationship between the number of foci formed in a BALB/c 3T3 transformation assay, and the number of genes, among the marker genes shown in generic group A, whose expression levels are up-regulated by more than 1.5-fold compared to the negative control, as determined by DNA microarray assay. When two or more of the genes are expressed at levels up-regulated by more than 1.5-fold compared to the negative control, the number of foci increases, and the test substance is evaluated as having tumor-promoting activity.
[FIG. 2]
   Fig. 2 shows a relationship between the number of foci formed in a BALB/c 3T3 transformation assay, and the sum of the expression levels of the marker genes shown in generic group A relative to the negative control, as determined by DNA microarray assay. When the sum of the expression levels relative to the negative control is 38 or more, the number of foci increases, and the test substance is evaluated as having tumor-promoting activity.
[Fig. 3]

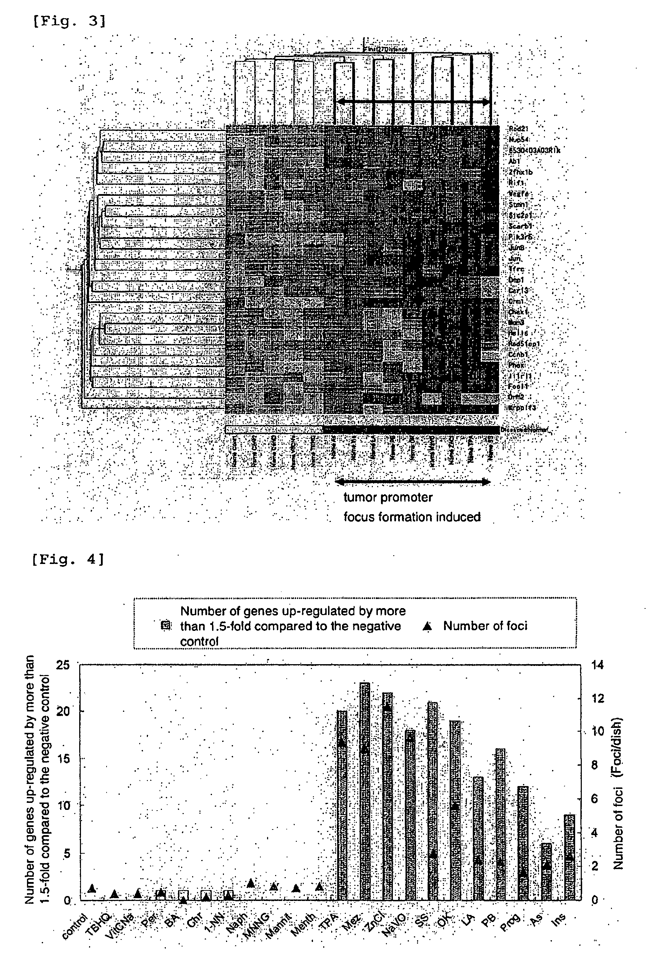

   Fig. 3 shows the results of cluster analysis using the marker genes of generic group A. Whether the test substance is a tumor promoter (shown by a bold line) can be determined from the genealogical tree shown at the top of Fig. 3.
[Fig. 4]
   Fig. 4 shows a relationship between the number of foci formed in a BALB/c 3T3 transformation assay, and the number of genes, among the marker genes shown in generic group A, whose expression levels are up-regulated by more than 1.5-fold compared to the negative control, as determined by quantitative RT-PCR. When two or more of the genes are expressed at levels up-regulated by more than 1.5-fold compared to the negative control, the number of foci increases, and the test substance is evaluated as having tumor-promoting activity.
[Fig. 5]

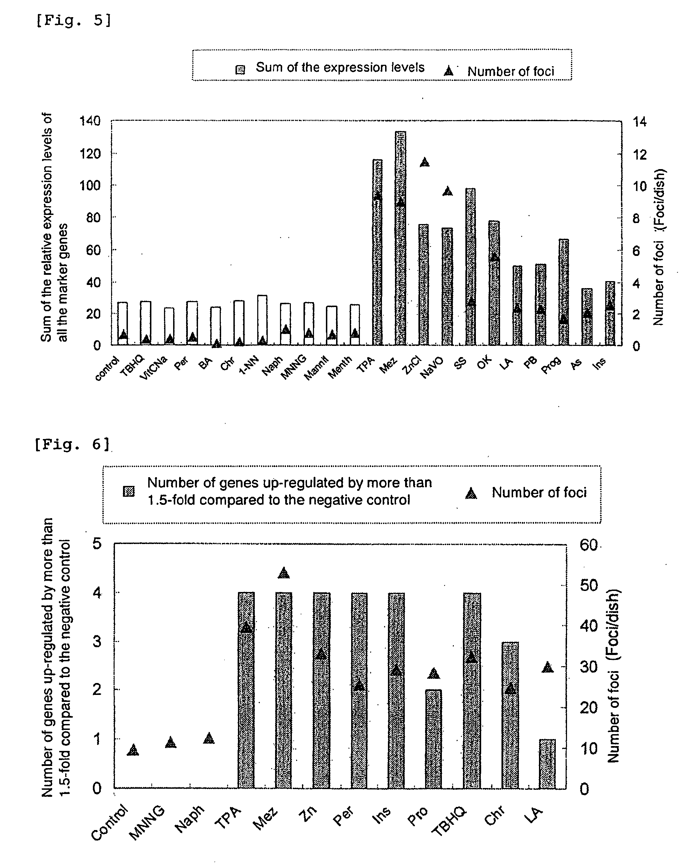

   Fig. 5 shows a relationship between the number of foci formed in a BALB/c 3T3 transformation assay, and the sum of the expression levels of the marker genes shown in generic group A relative to the negative control, as determined by quantitative RT-PCR. When the sum of the expression levels relative to the negative control is 40 or more, the number of foci increases, and the test substance is evaluated as having tumor-promoting activity.
[Fig. 6]
   Fig. 6 shows a relationship between the number of foci formed in a Bhas transformation assay, and the number of genes, among the 4 marker genes shown in generic group A, whose expression levels are up-regulated by more than 1.5-fold compared to the negative control, as determined by quantitative RT-PCR. When one or more of the genes are expressed at levels up-regulated by more than 1.5-fold compared to the negative control, the number of foci increases, and the test substance is evaluated as having tumor-promoting activity.
[Fig. 7]

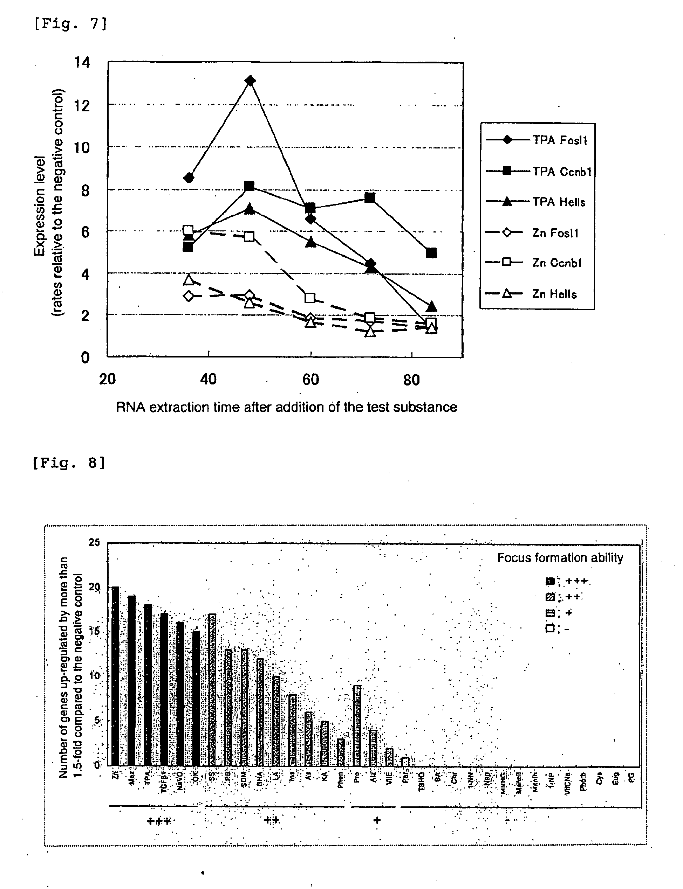

   Fig 7 shows the relationship between the RNA extraction time in a BALB/c 3T3 transformation assay using TPA or zinc chloride, and the expression levels of three of the marker genes for detection of tumor promoters shown in generic group A relative to the negative control, as determined by quantitative RT-PCR. 36 to 72 hours after the addition of TPA or zinc chloride, all three marker genes were expressed at levels up-regulated by more than 1.5-fold compared to the negative control.
[Fig. 8]
   Fig. 8 shows a relationship between the focus formation ability in a BALB/c 3T3 transformation assay, and the number of genes, among the marker genes shown in generic group A-3, whose expression levels are up-regulated by more than 1.5-fold compared to the negative control, as determined by quantitative RT-PCR. When two or more of the marker genes are expressed at levels up-regulated by more than 1.5-fold compared to the negative control, the focus formation ability increases, and the test substance is evaluated as having tumor-promoting activity.
[Fig. 9]

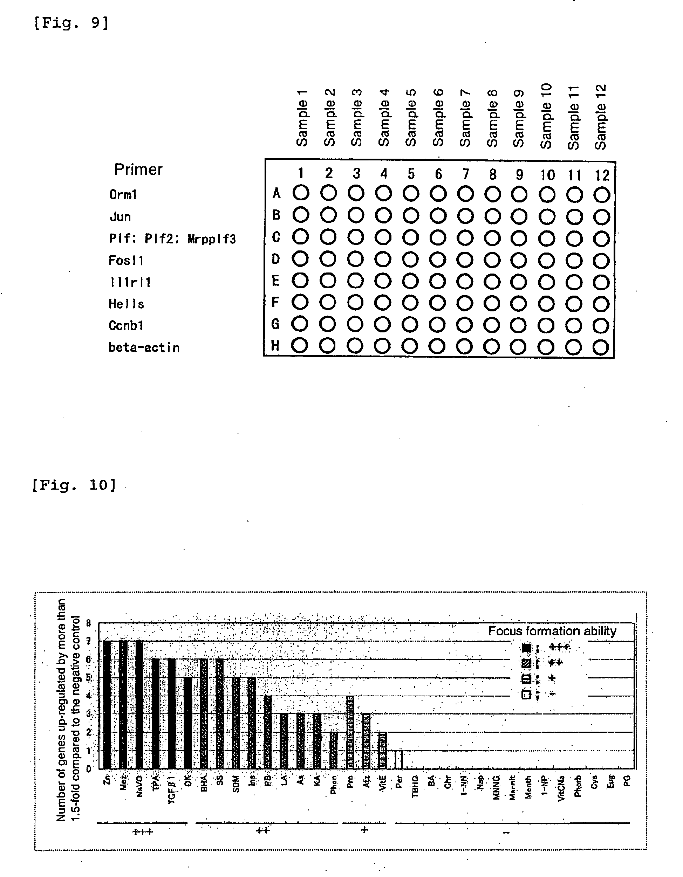

   Fig. 9 is a diagram of the arrangement of primers for the genes, and samples (cDNA produced by a reverse transcription reaction of RNA obtained 48 hours after the addition of each test substance) in a 96-well plate used in quantitative RT-PCR. Using one 96-well plate, the detection of tumor promoters from 12 samples can be performed using marker genes for detection of tumor promoters.
[Fig. 10]
   Fig. 10 shows a relationship between the focus formation ability in a BALB/c 3T3 transformation assay, and the number of genes, among 7 marker genes shown in generic group A-2, whose expression levels are up-regulated by more than 1.5-fold compared to the negative control, as determined by quantitative RT-PCR. When two or more of the marker genes are expressed at levels up-regulated by more than 1.5-fold compared to the negative control, the focus formation ability increases, and the test substance is evaluated as having tumor-promoting activity (+).
[Fig. 11]

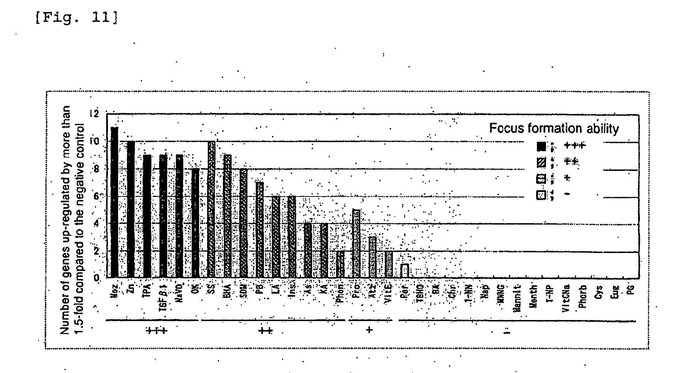

   Fig. 11 shows a relationship between the focus formation ability in a BALB/c 3T3 transformation assay, and the number of genes, among 11 marker genes shown in generic group A-2', whose expression levels are up-regulated by more than 1.5-fold compared to the negative control, as determined by quantitative RT-PCR. When two or more of the marker genes are expressed at levels up-regulated by more than 1.5-fold compared to the negative control, the focus formation ability increases, and the test substance is evaluated as having tumor-promoting activity.

### BEST MODE FOR CARRYING OUT THE INVENTION

The present invention will be described below in more detail with reference to Examples. However, the scope of the present invention is not limited to these Examples.

### Example 1

### Transformation assay using BALB/c 3T3 cells, and total RNA extraction

BALB/c 3T3 A31-1-1 cells (obtained from Japan Health Sciences Foundation) were seeded into 60 mm cell culture dishes (a product of Corning Incorporated) at a concentration of 10,000 cells/dish using MEM medium (manufactured by Nissui Pharmaceutical Co., Ltd.) containing 10% fetal bovine serum (FBS) (normal medium). Each test substance was added to twelve of the prepared 60 mm cell culture dishes in an amount of 5 mL/dish (day 0 after the start of test), and the cells were cultured at 37°C overnight. On the first day after the start of test, 3-methylcholanthlene, which is a tumor initiator, was added to the cells to a final concentration of 0.2 µg/mL, and the cells were cultured for 3 days. On the fourth day after the start of test, the cells were washed, the medium was replaced with normal medium, and the cells were further cultured for 3 days. On the seventh day after the start of the test, the medium was removed, and replaced with test substance-containing D-MEM/F-12 medium (GIBCO; a product of Invitrogen Corp.) containing 2% FBS and 0.2% ITS-X (GIBCO) (test medium). The following test substances were used as tumor promoters that were able to form foci in the BALB/c 3T3 cell transformation assay: 0.1 µg /mL of TPA, 7.5 µg/mL of zinc chloride, 1µg/mL of sodium orthovanadate, 5000 µg/mL of saccharin sodium, 0.0075 µg/mL of okadaic acid, 0.15 µg/mL of sodium arsenite, 30 µg/mL of insulin, 500 µg/mL of phenobarbital sodium, and 7.5 µgof lithocholic acid. As substances unable to form foci in the BALB/c 3T3 cell transformation assay, 100µg/mL of sodium ascorbate and 2 µg/mL of TBHQ (tert-butylhydroquinone) were used. As a negative control, a solvent alone was used. 48 hours after addition of each test substance, the medium was removed from one of the 12 dishes in each test substance-added group. After washing with PBS, the total RNA was extracted using an RNeasy Mini kit (manufactured by Qiagen Inc.) including a DNase step. 96 hours after addition of the test substance, the medium was removed from one of the remaining dishes, and the cells were stained with Crystal violet to determine the cytotoxicity. The remaining 10 dishes were used to perform a regular transformation assay using BALB/c 3T3 cells. On the twenty-fifth day after the start of test, the number of foci in the transformed cells was determined. When the number of foci formed in the test substance-added group was significantly increased compared to the negative control group (P<0.05, Wilcoxon (Mann-Whitney) test), the test substance was evaluated as having tumor-promoting activity.

### DNA microarray expression analysis of marker-gene

Using the total RNA obtained above, DNA microarray gene expression analysis was performed. In the DNA microarray experiment, GeneChip, Mouse Genome 430 2.0 Array available from Affymetrix, Inc., was used. For data analysis, GeneSpring GX ver.7.3.2 available from Agilent Technologies, Inc. was used. After normalization of DNA microarray data of each test substance-added group, the expression level of each of approximately 40,000 types of genes contained in the DNA microarray was divided by that of the negative control (a group that received only a solvent in the transformation assay using BALB/c 3T3 cells) to calculate the expression level relative to the negative control. From these approximately 40,000 genes, 27 types of marker genes for detection of tumor promoters shown in generic group A were selected according to the marker gene selection rules described above.

Among the 27 types of marker genes for detection of tumor promoters, the number of genes whose expression levels were up-regulated by more than 1.5-fold compared to the negative control was determined, and compared with the number of foci formed in the BALB/c 3T3 cell transformation assay (Fig. 1). Fig. 1 shows that the number of foci increases in proportion to the increase in the number of marker genes expressed up-regulated by more than 1.5-fold compared to the negative control. Accordingly, the results show that tumor promoters can be detected, and the intensity of the tumor-promoting activity can also be determined.

The sum of the relative expression levels of 27 marker genes obtained by DNA microarray analysis was compared with the number of foci in the BALB/c 3T3 cell transformation assay (Fig. 2). Fig. 2 shows that the number of foci increases in proportion to the increase in the sum of the relative expression levels of the marker genes. Accordingly, the results show that tumor promoters can be detected, and the intensity of the tumor-promoting activity can also be determined.

Fig. 3 shows the results of cluster analysis of 27 types of marker genes for detection of tumor promoters. Using GeneSpring GX ver.7.3.2 available from Agilent Technologies, Inc., the cluster analysis was performed under the following conditions: similarity measure; distance, clustering algorism; and average linkage. The genealogical tree in the upper portion of Fig. 3 shows that test substances having tumor promotion activity, which induced significant focus formation in the BALB/c 3T3 cell transformation assay, and test substances not having promoter activity, which did not induce focus formation, belong to different groups. Accordingly, whether the test substance is a tumor promoter can be determined from the genealogical tree.

### Example 2

A BALB/c 3T3 cell transformation assay was performed using the following compounds. The compounds used as tumor promoters that were able to form foci in the BALB/c 3T3 cell transformation assay were: 0.1µg/mL of TPA, 0.1µg/mL of mezerein, 7.5 µg/mL of zinc chloride, 1 µg/mL of sodium orthovanadate, 5000 µg/mL of saccharin sodium, 0.0075 µg/mL of okadaic acid, 7.5 µg/mL of lithocholic acid, 500 µg/mL of phenobarbital sodium, 2 µg/mL of progesterone, 0.15 µg/mL of sodium arsenite, and 30 µg/mL of insulin. The compounds used as substances that were unable to form foci in the BALB/c 3T3 cell transformation assay were 2 µg/mL of TBHQ, 100 µg/mL of sodium ascorbate, µg/mL of perylene, 5 µg/mL of benzo[a]anthracene, 1 µg/mL of chrysene, 10µg/mL of 1-nitronaphthalene, 3 µg/mL of naphthalene, 1 µg/mL of MNNG (N-methyl-N'-nitro-N-nitrosoguanidine), 300 µg/mL of D-mannitol, and 100 µg/mL of DL-menthol. As a negative control, a solvent alone was used. 48 hours after addition of each test substance, the medium was sampled from one of 12 dishes of each test substance-added group. After washing with PBS, the total RNA was extracted using a RNeasy Mini kit (manufactured by Qiagen Inc.) including a DNase step. One of the remaining dishes was used to determine the cytotoxicity, and the remaining ten dishes were used to determine the number of foci. When the number of foci formed in the test substance-added group was significantly increased compared to the negative control group (P<0.05, Wilcoxon (Mann-Whitney) test), the test substance was evaluated as having tumor-promoting activity.

### Quantitative RT-PCR expression analysis of marker-gene

Using the total RNA obtained in the above test, quantitative RT-PCR was performed using primers for marker genes for detection of tumor promoters shown in Table 2, and using a 7500 Real-Time PCR System available from Applied Biosystems. The PCR primers for detecting the marker genes are not limited to those of the sequences shown in Table 2. Any appropriately designed primer or commercially available primer can be used, as long as the primer can detect the expression of at least one of the marker genes. As a first step of quantitative RT-PCR, 100 ng of the total RNA obtained from each test substance-added group was subjected to a reverse transcription reaction using a High Capacity cDNA Reverse Transcription Kit available from Applied Biosystems, Inc. to produce cDNA. To each well of a PCR 96-well plate were added 1 µL of cDNA, 1.3 µL of 5 µM primers for one of the marker genes for detection of tumor promoters shown in Table 2, 10 µL of a Power SYBR Green PCR Master Mix available from Applied Biosystems, Inc., and 7.7 µL of ultrapure water. After incubation at 50°C for 2 minutes and at 95°C for 10 minutes, 35 to 40 cycles of PCR were performed using a 7500 Real-Time PCR System of Applied Biosystems, Inc., each cycle comprising 95°C for 15 seconds, and 60°C for 60 seconds. A dissociation curve analysis was then performed. A standard curve was prepared using a dilution series of cDNA of a negative or positive control, and the expression levels relative to the standard curve were calculated. The calculated expression levels were normalized by the expression levels of β-actin used as an internal standard gene. The normalized levels were divided by that of the negative control group. The expression level of each gene was obtained as an expression level relative to the negative control.

**[Table 2]**

| Reference sequence | Gene symbol | 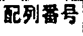 | Primer name | Primer sequence |
|---|---|---|---|---|
| NM_008768 | Orm1 | 1 | Orm1 primer sense | ACTCCACCCATCTAGGATTCCA |
| | | 2 | Orm1 primer antisense | GCAAAGGTTTCTACTCCTCCTTCA |
| NM_016741 | Scarb1 | 3 | Scarb1 primer sense | GCCAAGCTATAGGGTCCTGAAG |
| | | 4 | Scarb1 primer antisense | GACTGGGTGGCTGGTCTGA |
| NM_019641 | Stmn1 | 5 | Stmn1 primer sense | CCCACAAAATGGAGGCTAACA |
| | | 6 | Stmn1 primer antisense | TCCACGTGCTTGTCCTTCTCT |
| NM_009009 | Rad21 | 7 | Rad21 primer sense | GGTCTTCAGCGAGCTCTTGCTA |
| | | 8 | Rad21 primer antisense | CGACACAGCTCAAGCAAACTG |
| NM_183392 | Nup54 | 9 | Nup54 primer sense | AGATGCAGACCTGTTACGAGAAATC |
| | | 10 | Nup54 primer antisense | TCAAGTGGCTAAGGCCTTCCT |
| NM_010591 | Jun | 11 | Jun primer sense | ATTGCTTCTGTAGTGCTCCTTAACAC |
| | | 12 | Jun primer antisense | TGCAGTCTAGCCTGGCACTTAC |
| NM_016779 | Dmp1 | 13 | Dmp1 primer sense | CCAGAGGGACAGGCAAATAGTG |
| | | 14 | Dmp1 primer antisense | GCCCAGCTCCTCTCCAGATT |
| NM_001077190: NM_001077192; | Abi1 | 15 | Abi1 primer sense | AAAATTCTCTGACCTTTAATCCTATGGT |
| NM_001 077193 : NM_007380 : NM_145994 | | 16 | Abi1 primer antisense | TGCCCACATGTAAAGCCATTAC |
| NM_028382 | 6530403A03 Rik | 17 | 6530403A03 primer sense | ATTGAAAATGACAGTGACCTGTTTG |
| | | 18 | 6530403A03 primer antisense | GGACTTTTTCGGCTATTATCTTGATT |
| NM_011400 | Slc2a1 | 19 | Slc2a1 primer sense | TCCAACTGGACCTCAAACTTCA |
| | | 20 | Slc2a1 primer antisense | CCGCACAGTTGCTCCAGATA |
| NM_011118 ; | Plf ; Plf2 ; | 21 | Mrpplf3primer sense | GCCACAGACATAAAGAAAAAGATCAAC |
| NM_011954 ; NM_031191 | Mrpplf3 | 22 | Mrpplf3primer antisense | TCTTCTTTTCTTCATCTCCATTCTGA |
| NM_010235 | Fosl1 | 23 | Fosl1 primer sense | CCGAAGAAAGGAGCTGACAGA |
| | | 24 | Fosl1 primer antisense | CGATTTCTCATCCTCCAATTTGT |
| NM_007691 | Chek1 | 25 | Chek1 primer sense | CCGACTTTCTAAGGGTGATGGA |
| | | 26 | Chek1 primer antisense | CGCTGAGCTTCCCTTTAATCTTC |
| NM_177320 | Pik3r5 | 27 | Pik3r5 primer sense | GCAGAGTGTGGTCAGGTGTGA |
| | | 28 | Pik3r5 primer antisense | GGTGGCAAGCTGCTCTTCTC |
| NM_008416 | Junb | 29 | Junb primer sense | GCCCTGGCAGCCTGTCT |
| | | 30 | Junb primer antisens | GCGCCAAGGTGGGTTTC |
| NM_001025250; | Vegfa | 31 | Vegfa primer sense | TGCACCCACGACAGAAGGA |
| NM_001025257; NM_009505 | | 32 | Vegfa primer antisense | TCGCTGGTAGACATCCATGAAC |
| NM_175238 ; | Rif1 ; | 33 | Rif1 primer sense | CAGGACTGTCTCCACGGATGA |
| XR_003484 | LOC671598 | 34 | Rif1 primer antisense | GGGTATCTAGGGTCACAGGTTCA |
| NM_001025602 : | Il1rl1 | 35 | Il1rl1 primer sense | CTGCAGGAAAAGAGAATCCAAAC |
| NM_010743 | | 36 | Il1rl1 primer antisense | GGAAGGCATTGTGGAATCAAG |
| NM_011077 | Phex | 37 | Phex primer sense | GCCAAGAGAAATGGGAAAGCT |
| | | 38 | Phex primer antisense | AGCACAAAACCTGTCCTTCCA |
| NM_011638 | Tfro | 39 | Tfrc primer sense | TTGAGGCAGACCTTGCACTCT |
| | | 40 | Tfro primer antisense | AAAGCCAGGTGTGTATGGATCA |
| NM_015753 | Zfhx1b | 41 | Zfhx1b primer sense | GTGACAAGACATTCCAGAAAAGCA |
| | | 42 | Zfhx1b primer antisense | TGGTGTGGTCTCTTTCCTGTGT |
| NM_009013 | Rad51ap1 | 43 | Red51ap1 primer sense | TGAAAGCAAGAGGCCCAAGT |
| | | 44 | Rad51ap1 primer antisense | AATGCATTGCTGCTAGAGTTGCT |
| NM_008234 | Hells | 45 | Hells primer sense | TCTAGAATTACTGTTGGATCGAAGTGA |
| | | 46 | Hells primer antisense | TCCCTGTCTTCCCTTTAATTGG |
| NM_008583 | Mcm3 | 47 | Mcm3 primer sense | CCCAGGACTCCCAGAAAGTG |
| | | 48 | Mcm3 primer antisense | GAGGGCCGCCTTAAAAGC |
| NM_011016 | Orm2 | 49 | Orm2 primer sense | ACCTTACCCCCAACTTGATAAATG |
| | | 50 | Orm2 primer antisense | ACAGTGGTCATCTATGGTGTGATACTC |
| NM_024495 | Car13 | 51 | Car13 primer sense | TTGAGAGTGTCACGTGGATTGTT |
| | | 52 | Car13 primer antisense | CACAAGAGGCTTCGGAATCTG |
| NM_172301 | Ccnb1 | 53 | Ccnb1 primer sense | GCAGCACGTGGTAAGAATGT |
| | | SEQ ID NO | Ccnb1 primer antisense | TTCTTGACAGTCATGTGCTTTGTG |

The number of marker genes whose expression levels are up-regulated by more than 1.5-fold compared to the negative control was determined, and compared with the number of foci formed in a BALB/c 3T3 cell transformation assay (Fig. 4). Fig. 4 shows that the number of foci increases in proportion to an increase in the number of marker genes up-regulated by more than 1.5-fold compared to the negative control. Accordingly, the tumor promoters can be detected, and the intensity of the tumor-promoting activity can also be determined therefrom.

The sum of the relative expression levels of marker genes obtained by quantitative RT-PCR was compared with the number of foci formed in a BALB/c 3T3 cell transformation assay (Fig. 5). Fig. 5 shows that the number of foci increases in proportion to an increase in the sum of the relative expression levels of the marker genes. Accordingly, the results show that tumor promoters can be detected and the intensity of the tumor-promoting activity can also be determined.

### Example 3

Quantitative RT-PCR was.performed using the RNA obtained in Example 2 to determine the expression level of the Orm1 gene. The conditions for the quantitative RT-PCR were the same as in Example 2. As shown in Table 3 below, when the expression level of Orm1 is up-regulated by more than 1.5-fold compared to the negative control, significant focus formation in the transformed cells can be predicted. When the number of foci formed in the test substance-added group was significantly increased compared to the negative control (P<0.05, Wilcoxon (Mann-Whitney) test), the test substance was evaluated as inducing significant focus formation (having tumor-promoting activity).

**[Table 3]**

| Test substance | Expression level of Orm1 (rates relative to the negative control) | Focus formation in the BALB/c 3T3 cell transformation assay * |
|---|---|---|
| TBHQ | 0.9 | - |
| Sodium ascorbate (VitCNa) | 0.6 | - |
| Perylene (Per) | 0.6 | - |
| Benz[a]anthracen (BA) | 1.1 | - |
| Chrysene (Chr) | 1.4 | - |
| 1-nitronaphthalene (1-NN) | 1.0 | - |
| Naphthalene (Naph) | 1.3 | - |
| MNNG | 1.3 | - |
| D-mannitol (Mannit) | 0.9 | - |
| dl-menthol(Menth) | 0.9 | - |
| TPA | 2.3 | + |
| Mezerein (Mez) | 2.6 | + |
| Zinc chloride (ZnCl) | 3.4 | + |
| Sodium orthovanadate (NaVO) | 1.5 | + |
| Saccharin sodium (SS) | 3.2 | + |
| Okadaic acid (OK) | 8.0 | + |
| Lithocholic acid (LA) | 5.8 | + |
| Phenobarbital sodium (PB) | 7.2 | + |
| Progesterone (Prog) | 18.7 | + |
| Sodium arsenite (As) | 1.7 | + |
| Insulin (Ins) | 2.0 | + |

| | | |
|---|---|---|
| *) **-: no significant focus formation induced;** **+: significant focus formation induced** | | |

### Example 4

### Tumor promoter detection in a transformation assay using Bhas cells

Bhas cells (obtained from Japan Health Sciences Foundation) were seeded into 6-well cell culture plates (#3910, manufactured by Corning Incorporated) to a concentration of 20,000 cells/mL using D-MEM/F-12 medium containing 5% FBS. Each test substance was added to 7 wells in an amount of 2 mL/well (on day 0 after the start of the test), and cultured at 37°C for 3 days. On the third day after the start of the test, the medium was replaced with test substance-containing D-MEM/F-12 medium containing 2% FBS. With respect to the test substances, the following compounds were used as tumor promoters that were able to form foci in the Bhas cellular transformation assay: 0.05 µg/mL of TPA, 0.001 µg/mL of mezerein, 10 µg/mL zinc chloride, 1 µg/mL of perylene, 50 µg/mL of insulin, 10 µg/mL of progesterone, 5 µg/mL of TBHQ, 1 µg/mL of chrysene, and 5 µg/mL of lithocholic acid. As non-tumor-promoting substances that were unable to form foci in the Bhas cellular transformation assay, 0.1 µg/mL of MNNG and 10 µg/mL of naphthalene were used. As a negative control, a solvent alone was used. 48 hours after addition of the test substance, the medium was removed from one of the wells of each test substance-added group. After washing with PBS, the total RNA was extracted using an RNeasy Mini kit (manufactured by Qiagen, Inc.) including a DNase step. Six wells each of the remaining wells were used to perform a regular transformation assay using Bhas cells. On the twenty-first day after the start of the test, the number of foci of the transformed cells was determined.

For expression analysis of the marker genes for detection of tumor promoters, the total RNA obtained in the transformation assay using Bhas cells was subjected to quantitative RT-PCR using 4 promoters, Ccnb1, Hells, Rad51ap1, and Fosl1 selected from the marker genes for detection of tumor promoters shown in Table 2, and using a 7500 Real-Time PCR System manufactured by Applied Biosystems, Inc. The expression levels of the marker genes for detection of tumor promoters were normalized by the expression level of β-actin used as an internal standard gene. The normalized expression levels were divided by the expression level of the negative control group. The expression level of each of the 4 genes was obtained as a expression level relative to that of the negative control group. The conditions for the quantitative RT-PCR were the same as in Example 2. Among the 4 genes, the number of marker genes whose expression levels were up-regulated by more than 1.5-fold compared to the negative control was determined, and compared with the number of foci formed in the Bhas cellular transformation assay (Fig. 6). Fig. 6 shows that the number of foci increases in proportion to the increase in the number of marker genes. Accordingly, the results show that tumor promoters can be detected, and the intensity of the tumor-promoting activity can also be determined.

### Example 5

### Analysis of RNA extraction time

A BALB/c 3T3 cell transformation assay was performed in the same manner as in Example 1 using 0.1 µg/mL of TPA or 7.5 µg/mL of zinc chloride as a test substance. 36, 48, 60, 72, and 80 hours after addition of the test substance, the medium was removed from one of the 15 dishes of each test substance-added group. After washing with PBS, the total RNA was extracted using an RNeasy Mini kit (manufactured by Qiagen, Inc.) including a DNase step. One of the remaining dishes was used to determine the cytotoxicity. Ten of the remaining dishes were used to perform a transformation assay using BALB/c 3T3 cells. On the twenty-fifth day after the start of the test, the number of foci of the transformed cells was determined. Significant focus formation was observed both in the TPA-added group and in the zinc chloride-added group.

For expression analysis of the marker genes for detection of tumor promoters, the total RNA obtained in the above test was subjected to quantitative RT-PCR using 3 primers, Ccnb1, Hells, and Fosll, among the marker genes for detection of marker promoters shown in Table 2, and using a 7500 Real-Time PCR System manufactured by Applied Biosystems, Inc. The expression level of each of the marker genes for detection of tumor promoters was normalized by the expression level of β-actin used as an internal standard gene. The normalized expression level was divided by the expression level of the negative control group. The expression level of each of the 3 genes was obtained as a relative expression level to that of the negative control group. The conditions for the quantitative RT-PCR were the same as in Example 2.

Fig. 7 shows the relationship between the expression levels of the 3 genes, and the RNA extraction time after addition of the test substance. The results show that the expression levels of these marker genes in RNA extracted 36 hours to 72 hours after addition of the test substance are up-regulated by more than 1.5-fold compared to the negative control, and tumor promoters can be detected.

### Example 6

### Quantitative RT-PCR expression analysis using 22 types of marker genes

A BALB/c 3T3 cell transformation assay was performed in the same manner as in Example 1 using the following 33 substances as test compounds: 0.1 µg/mL of TPA, 0.1 µg/mL of mezerein, 7.5 µg/mL of zinc chloride, 1 µg/mL of sodium orthovanadate, 5000 µg/mL of saccharin sodium, 0.0075 µg/mL of okadaic acid, 7.5 µg/mL of lithocholic acid, 500 µg/mL of phenobarbital sodium, 2 µg/mL of progesterone, 0.15 µg/mL of sodium arsenite, 30 µg/mL of insulin, 0.003 µg/mL of a transforming growth factor, 100 µg/mL of sulfadimethoxine, 100 µg/mL of Kojic acid, 30 µg/mL of butylhydroxyanisol, 30 µg/mL of atrazine, 3 µg/mL of DL-α-tocopherol, 3 µg/mL of phenacetin, 2 µg/mL of TBHQ, 100 µg/mL of sodium ascorbate, 5 µg/mL of benzo[a]anthracene, 1 µg/mL of chrysene, 10 µg/mL of 1-nitronaphthalene, 3 µg/mL of naphthalene, 1 µg/mL of MNNG (N-methyl-N'-nitro-N-nitrosoguanidine), 300 µg/mL of D-mannitol, 100 µg/mL of DL-menthol, 1 µg/mL of 1-nitropyrene, 1 µg/mL of phorbol, 3 µg/mL of eugenol, 1 µg/mL of propyl gallate, 1 µg/mL of perylene, and 100 µg/mL of L-cysteine hydrochloride. 48 hours after addition of the test substance, the medium was removed from one of the 12 dishes of each test substance-added group. After washing with PBS, the total RNA was extracted using an RNeasy Mini kit (manufactured by Qiagen, Inc.). One of the remaining dishes was used to determine the cytotoxicity. Ten of the remaining dishes were used to perform a transformation assay using BALB/c 3T3 cells. On the twenty-fifth day after the start of the test, the number of foci of the transformed cells was determined. Test substances were simply classified into 3 groups using the focus formation ability (intensity of the tumor-promoting activity) as an index.
When the number of foci was significantly increased compared to the negative control (P<0.05, Wilcoxon (Mann-Whitney) test) and was at least 50% that of the positive control TPA, a group of such test substances were evaluated as having potent tumor-promoting activity (+++). The test substances evaluated as +++ were TPA, mezerein, zinc chloride, sodium orthovanadate, okadaic acid, and transforming growth factors. When the number of foci was significantly increased compared to the negative control (P<0.05, Wilcoxon (Mann-Whitney) test) and was less than 50% that of the positive control TPA, a group of such test substances were evaluated as having tumor-promoting activity (++). The test substances evaluated as "++" were lithocholic acid, phenobarbital sodium, sodium arsenite, saccharin sodium, sulfadimethoxine, kojic acid, insulin, butylhydroxyanisol, and phenacetin. When the number of foci was increased at least 2-fold compared to the negative control (P<0.05, Wilcoxon (Mann-Whitney) test) but the difference is not significant (P<0.05), a group of such test substances were evaluated as having weak tumor-promoting activity (+). The test substances evaluated as "+" were progesterone, atrazine, and DL-α-tocopherol. All the other test substances were evaluated as having no tumor promoting activity (-), because the number of foci did not increase 2-fold or more compared to that of the negative control, and no significant changes were observed in the number of foci.

For expression analysis of the marker genes for detection of tumor promoters, the total RNA obtained in the above test was subjected to quantitative RT-PCR using primers shown in Table 2 corresponding to the 22 marker genes for detection of tumor promoters shown in generic group A-3, and using a 7500 Real-Time PCR System of Applied Biosystems, Inc. The expression level of each of the marker genes for detection of tumor promoters was normalized by the expression level of β-actin used as an internal standard gene. The normalized expression level was divided by the expression level of the negative control group. The expression level of each of the 22 genes was obtained as an expression level relative to that of the negative control group. The conditions for the quantitative RT-PCR were the same as in Example 2.

The number of marker genes whose expression levels were up-regulated by more than 1.5-fold compared to the negative control was determined, and compared with focus formation ability in the BALB/c 3T3 cell transformation assay (Fig. 8). Fig. 8 shows that the focus formation ability increases in proportion to the increase in the number of marker genes whose expression levels were up-regulated by more than 1.5-fold compared to the negative control. Accordingly, the results show that tumor promoters can be detected, and the intensity of the tumor-promoting activity can also be easily determined from the number of marker genes whose expression levels are up-regulated by more than 1.5-fold compared to the negative control.

### Example 7

### Quantitative RT-PCR expression analysis using 7 types of marker genes

The RNA obtained in Example 6 was subjected to quantitative RT-PCR to determine the expression levels of 7 marker genes for detection of tumor promoters (Orml; NM_008768, Jun; NM_010591 and Plf; Plf2; Mrpplf3; NM_011118; NM_011954; NM_031191, Fosl1; NM_010235, Il1rl1; NM_001025602; NM_010743, Hells; NM_008234, Ccnb1; NM_172301). More specifically, primers for the 7 genes were respectively added to rows A, B, C, D, E, F, and G of a PCR 96-well plate, and primers for β-actin used as an internal standard gene were added to row H. Then cDNA obtained from each sample was added to columns 1 to 12 of the 96-well plate in an amount of 1 µL/well (12 samples can be tested per 96-well plate) (Fig. 9). The conditions for the quantitative RT-PCR were the same as in Example 2.

The number of marker genes whose expression levels were up-regulated by more than 1.5-fold compared to the negative control was determined, and compared with the focus formation ability in the BALB/c 3T3 cell transformation assay (Fig. 10). Fig. 10 shows that the focus formation ability increases in proportion to the increase in the number of marker genes up-regulated by more than 1.5-fold compared to the negative control. Accordingly, the results show that tumor promoters can be detected, and the intensity of the tumor-promoting activity can also be determined from the number of marker genes up-regulated by more than 1.5-fold compared to the negative control.

### Example 8

### Quantitative RT-PCR expression analysis using 11 types of marker genes

Quantitative RT-PCR was performed using RNA obtained in Example 6 to determine the expression levels of 11 types of marker genes for detection of tumor promoters (Orm1; NM_008768, Jun; NM_010591, Plf; Plf2; Mrpplf3; NM_011118; NM_011954; NM_031191, Fosl1; NM_010235, Il1rl1; NM_001025602; NM_010743, Hells; NM_008234, Ccnb1; NM_172301, Slc2al; NM_011400, Phex; NM_011077, Scarb1; NM_016741, Vegfa; NM_001025250; NM_001025257; NM_009505). More specifically, primers for the 11 genes were respectively added to column 1 to 11 of a PCR 96-well plate, and primers for β-actin used as an internal standard gene were added to column 12. cDNA obtained from each sample was added to rows A to H of the 96-well plate in an amount of 1 µL/well (8 samples can be tested per 96-well plate). The conditions for the quantitative RT-PCR were the same as in Example 2.

The number of marker agents whose expression levels were up-regulated by more than 1.5-fold compared to the negative control was determined, and compared with the focus formation ability in the BALB/c 3T3 cell transformation assay (Fig. 11). Fig. 11 shows that the focus formation ability increases in proportion to the increase in the number of marker genes up-regulated by more than 1.5-fold compared to the negative control. Accordingly, the results show that tumor promoters can be detected, and the intensity of the tumor-promoting activity can also be determined from the number of marker genes up-regulated by more than 1.5-fold compared to the negative control.

## Claims

1. Use of a set of marker-genes as a marker in a method of detecting a tumor promoting agent using as BALB/c 3T3 cell, a clonal strain thereof, or a transformant thereof, the set of marker-genes comprising at least 7 genes in the following genetic group A-2:
(Gene Symbol)
Orm1
Jun
Plf ; Plf2 ; Mrpplf3.
Fosl1
Il1rl1
Hells
Ccnb1
wherein the three genes Plf, Plf2 and Mrpplf3 are assayed as identical.

2. The use of claim 1, wherein the set of marker-genes comprising at least 11 genes in the following genetic group A-2':
(Gene symbol)
Orm1
Jun
Plf ; Plf2 ; Mrpplf3
Fossl1
Il1rl1
Hells
Ccnb1
Slc2a1
Phex
Scarb1
Vegfa

3. The use of claim 1 or 2, wherein the set of marker-genes comprising at least 22 genes in the following genetic group A-3:
(Gene Symbol)
Orm1
Scarb1
Stmn1
Nup54
Jun
Abi1
Slc2a1
Plf ; Plf2 ; Mrpplf3
Fosl1
Chek1
Pik3r5
Vegfa
Rif1 ; LOC671598
Il1rl1
Phex
Tfrc
Rad51ap1
Hells
Mcm3
Orm2
Car13
Ccnb1

4. A method of detecting a tumor promoting agent, comprising the steps of:
bringing a BALB/c 3T3 cell, a clonal strain thereof, or a transformant thereof into contact with a test substance;
determining the expression level of a marker-gene in the cell brought into contact with the test substance;
comparing the determined expression level with the expression level of a control brought into contact with a test substance-free solvent; and
evaluating the test substance as having tumor-promoting activity, when the comparison shows that (i) the sum of a set of marker-genes expression levels or (ii) the number of genes of a set of marker-genes expressed at high levels in the test substance-contacted cells is greater than that of the control,
the set of marker-genes being a set of marker-genes defined in one of claims 1 to 3.

5. Use of a kit in a method of detecting a tumor promoting agent using a BALB/c 3T3 cell, a clonal strain thereof, or a transformant thereof, the kit comprising a reagent for determining the set of marker-genes of any one of claims 1 to 3, wherein said reagent is selected from the group consisting of
(a) a reagent for measuring the expression levels by PCR comprising a sense or antisense primer or one or more sets of sense and antisense primers for each marker gene of any one of claims 1 to 3;
(b) a reagent for northern blotting assay comprising probes for the marker genes of any one of claims 1 to 3;
(c) a reagent for DNA microarray assay comprising arrays carrying probes for the marker genes of any one of claims 1 to 3; and
(d) a reagent for immunoassay comprising antibodies to transcripts of the marker genes of any one of claims 1 to 3 or microplates on which said antibodies are immobilized.

## Patentansprüche

1. Verwendung eines Sets von Markergenen als Marker in einem Verfahren zum Nachweis eines tumorfördernden Agens unter Verwendung einer BALB/c 3T3-Zelle, eines clonalen Stamms davon, oder einer Transformante davon, wobei das Set von Markergenen mindestens 7 Gene der folgenden genetischen Gruppe A-2 umfasst:
(Gensymbol)
Orm1
Jun
Plf; Plf2; Mrpplf3
Fosl1
Il1rl1
Hells
Ccnb1
wobei die drei Gene Plf, Plf2 und Mrpplf3 als identisch getestet werden.

2. Verwendung nach Anspruch 1, wobei das Set von Markergenen mindestens 11 Gene der folgenden gentischen Gruppe A-2' umfasst:
(Gensymbol)
Orm1
Jun
Plf; Plf2; Mrpplf3
Fosl1
Il1rl1
Hells
Ccnb1
Slc2a1
Phex
Scarb1
Vegfa

3. Verwendung nach Anspruch 1 oder 2, wobei das Set von Markergenen mindestens 22 Gene der folgenden genetischen Gruppe A-3 umfasst:
(Gensymbol)
Orm1
Scarb1
Stmn1
Nup54
Jun
Abi1
Scl2a1
Plf; Plf2; Mrpplf3
Fosl1;
Chek1
Pik3r5
Vegfa
Rif1; LOC671598
Il1rl1
Phex
Tfrc
Rad51ap1
Hells
Mcm3
Orm2
Car13
Ccnb1

4. Verfahren zum Nachweis eines tumorfördernden Agens, umfassend die Schritte:
Inkontaktbringen einer BALB/c 3T3-Zelle, eines clonalen Stamms davon oder einer Transformante davon mit einer Testsubstanz;
Bestimmen des Expressionspiegels eines Markergens in der Zelle, die mit der Testsubstanz in Kontakt gebracht wurde;
Vergleichen des bestimmten Expressionsspiegels mit dem Expressionsspiegel einer Kontrolle, die mit einem Testsubstanz-freien Lösungsmittel in Kontakt gebracht wurde; und
Bewerten, dass die Testsubstanz tumorfördernde Aktivität aufweist, wenn der Vergleich zeigt, dass (i) die Summe der Expressionsspiegel eines Sets von Markergenen oder (ii) die Anzahl der Gene eines Sets von Markergenen, die in hohen Spiegeln in den mit der Testsubstanz in Kontakt gebrachten Zellen exprimiert werden, höher ist als die der Kontrolle,
wobei das Set von Markergenen ein Set von Markergenen wie in einem der Ansprüche 1 bis 3 definiert ist.

5. Verwendung eines Kits in einem Verfahren zum Nachweis eines tumorfördernden Agens unter Verwendung einer BALB/c 3T3-Zelle, eines clonalen Stamms davon oder einer Transformante davon, wobei der Kit ein Reagenz zum Bestimmen des Sets von Markergenen nach einem der Ansprüche 1 bis 3 umfasst, wobei das Reagenz ausgewählt ist aus der Gruppe bestehend aus
(a) einem Reagenz zum Messen der Expressionsspiegel mittels PCR, umfassend einen Sense- oder Antisense-Primer oder ein oder mehrere Sets von Sense- und Antisense-Primern für jedes Markergen nach einem der Ansprüche 1 bis 3;
(b) einem Reagenz für einen Northern-Blot-Assay, umfassend Sonden für die Markergene nach einem der Ansprüche 1 bis 3;
(c) einem Reagenz für einen DNA-Microarray-Assay, umfassend Arrays, die Sonden für die Markergene nach einem der Ansprüche 1 bis 3 tragen; und
(d) einem Reagenz für einen Immunassay, umfassend Antikörper gegen Transkripte der Markergene nach einem der Ansprüche 1 bis 3 oder Mikrotiterplatten, auf denen die Antikörper immobilisiert sind.

## Revendications

1. Utilisation d'un ensemble de gènes marqueurs en tant que marqueur dans un procédé de détection d'un agent promoteur de tumeur utilisant une cellule BALB/c 3T3, une souche clonale de celle-ci, ou un transformant de celle-ci, l'ensemble de gènes marqueurs comprenant au moins 7 gènes dans le groupe génétique A-2 suivant :
(Symbole du gène)
Orm1
Jun
Plf ; Plf2 ; Mrpplf3
Fosll
Il1rl1
Hells
Ccnb1
où les trois gènes Plf, Plf2 et Mrpplf3 sont analysés comme identiques.

2. Utilisation selon la revendication 1, dans laquelle l'ensemble de gènes marqueurs comprend au moins 11 gènes dans le groupe génétique A-2' suivant :
(Symbole du gène)
Orm1
Jun
Plf ; Plf2 ; Mrpplf3
Fosl1
Il1rl1
Hells
Ccnb1
Slc2a1
Phex
Scarb1
Vegfa

3. Utilisation selon la revendication 1 ou 2, dans laquelle l'ensemble de gènes marqueurs comprend au moins 22 gènes dans le groupe génétique A-3 suivant :
(Symbole du gène)
Orm1
Scarb1
Stmn1
Nup54
Jun
Abi1
Slc2al
Plf ; Plf2 ; Mrpplf3
Fosl1
Chek1
Pik3r5
Vegfa
Rif1 ; LOC671598
Il1rl1
Phex
Tfrc
Rad51ap1
Hells
Mcm3
Orm2
Car13
Ccnb1

4. Procédé de détection d'un agent promoteur de tumeur, comprenant les étapes qui consistent à :
mettre en contact une cellule BALB/c 3T3, une souche clonale de celle-ci, ou
un transformant de celle-ci, avec une substance de test ;
déterminer le taux d'expression d'un gène marqueur dans la cellule mise en contact avec la substance de test ;
comparer le taux d'expression déterminé avec le taux d'expression d'un contrôle mis en contact avec un solvant dépourvu de substance de test ; et
évaluer que la substance de test possède une activité promotrice de tumeur lorsque la comparaison montre que (i) la somme d'un ensemble de taux d'expression de gènes marqueurs ou (ii) le nombre de gènes d'un ensemble de gènes marqueurs exprimés à des taux élevés dans les cellules mises en contact avec la substance de test est plus élevé(e) que celle/celui du contrôle,
l'ensemble de gènes marqueurs étant un ensemble de gènes marqueurs défini dans l'une des revendications 1 à 3.

5. Utilisation d'un kit dans un procédé de détection d'un agent promoteur de tumeur utilisant une cellule BALB/c 3T3, une souche clonale de celle-ci, ou un transformant de celle-ci, le kit comprenant un réactif pour déterminer l'ensemble de gènes marqueurs selon l'une quelconque des revendications 1 à 3, ledit réactif étant sélectionné dans le groupe consistant en
(a) un réactif pour mesurer les taux d'expression par PCR comprenant une amorce sens ou antisens ou un ou plusieurs ensembles d'amorces sens et antisens pour chaque gène marqueur selon l'une quelconque des revendications 1 à 3 ;
(b) un réactif pour réaliser un essai de transfert de Northem comprenant des sondes pour les gènes marqueurs selon l'une quelconque des revendications 1 à 3 ;
(c) un réactif pour réaliser un essai de microréseau d'ADN comprenant des réseaux comportant des sondes pour les gènes marqueurs selon l'une quelconque des revendications 1 à 3 ; et
(d) un réactif pour réaliser un dosage immunologique comprenant des anticorps dirigés contre des transcrits des gènes marqueurs selon l'une quelconque des revendications 1 à 3 ou des microplaques sur lesquelles lesdits anticorps sont immobilisés.
